# EUROPEAN PATENT SPECIFICATION

(11) **EP 2 498 774 B1**
(45) Date of publication and mention of the grant of the patent: **11.10.2017**
(21) Application number: 10859431.8
(22) Date of filing: 12.11.2010
(51) Int. Cl.: C07H 19/167, A61K 31/426, A61K 31/56, A61P 11/06

(54) **DUAL-ACTION COMPOUNDS TARGETING ADENOSINE A2A RECEPTOR AND ADENOSINE TRANSPORTER FOR PREVENTION AND TREATMENT OF NEURODEGENERATIVE DISEASES**
AUF DEN ADENOSIN-A2A-REZEPTOR ZIELENDE DOPPELT WIRKENDE VERBINDUNGEN SOWIE ADENOSINTRANSPORTER ZUR VERHINDERUNG UND BEHANDLUNG NEURODEGENERATIVER ERKRANKUNGEN
COMPOSÉS À DOUBLE ACTION CIBLANT LE RÉCEPTEUR D'ADÉNOSINE A2A ET UN TRANSPORTEUR D'ADÉNOSINE POUR LA PRÉVENTION ET LE TRAITEMENT DE MALADIES NEURODÉGÉNÉRATIVES

(30) Priority: 13.11.2009 US 260932 P
(43) Date of publication of application: 19.09.2012
(73) Proprietor: Academia Sinica, Nan-Kang, Taipei (TW)
(72) Inventor: LIN, Yun-Lian, Taipei 10678 (TW); CHERN, Yijuang, Taipei 11529 (TW); FANG, Jim-Min, Taipei 106 (TW); LIN, Jung-Hsing, Taipei (TW); HUANG, Nai-Kuei, Taipei 114 (TW)
(74) Representative: Gill, Siân Victoria
(86) International application number: PCT/US2010/056516
(87) International publication number: WO 2012/064340

(56) References cited:
- WO-A2-2008/156513
- US-A1- 2003 225 205
- US-A1- 2007 237 840
- US-A1- 2007 249 638
- US-A1- 2008 064 653
- US-A1- 2008 132 525
- US-A1- 2008 176 816
- KUSACHI S ET AL: "DOG CORONARY ARTERY ADENOSINE RECEPTOR: STRUCTURE OF THE N6-ARYL SUBREGION", JOURNAL OF MEDICINAL CHEMISTRY, AMERICAN CHEMICAL SOCIETY, US, vol. 29, no. 6, 1 January 1986 (1986-01-01), pages 989-996, XP001147840, ISSN: 0022-2623, DOI: 10.1021/JM00156A016
- BRESSI J C ET AL: "ADENOSINE ANALOGUES AS INHIBITORS OF TRYPANOSOMA BRUCEI PHOSPHOGLYCERATE KINASE: ELUCIDATION OF A NOVEL BINDING MODE FOR A 2-AMINO-N6-SUBSTITUTED ADENOSINE", JOURNAL OF MEDICINAL CHEMISTRY, AMERICAN CHEMICAL SOCIETY, US, vol. 43, no. 22, 1 January 2000 (2000-01-01), pages 4135-4150, XP000999137, ISSN: 0022-2623, DOI: 10.1021/JM000287A
- AFIFY H M N M ET AL: "A NOVEL AND FACILE REACTION TO N6-ALKYLATED ADENOSINE VIA BENZOTRIAZOLE AS A SYNTHETIC AUXILIARY", JOURNAL OF HETEROCYCLIC CHEMISTRY, WILEY-BLACKWELL PUBLISHING, INC, US, vol. 37, no. 2, 1 March 2000 (2000-03-01), pages 339-341, XP001147847, ISSN: 0022-152X, DOI: 10.1002/JHET.5570370218
- UEEDA M ET AL: "2-Alkoxyadenosines: Potent and selective agonists at the coronary artery A2 adenosine receptor", JOURNAL OF MEDICINAL CHEMISTRY, AMERICAN CHEMICAL SOCIETY, US, vol. 34, 1 January 1991 (1991-01-01), pages 1334-1339, XP002225574, ISSN: 0022-2623, DOI: 10.1021/JM00108A014
- GALLO-RODRIGUEZ C ET AL: "Structure Activity Relationships of N6-Benzyladenosine-5'-uronamides as A3 Selective Adenosine Agonists", JOURNAL OF MEDICINAL CHEMISTRY, AMERICAN CHEMICAL SOCIETY, US, vol. 37, no. 5, 4 March 1994 (1994-03-04), pages 636-646, XP002136400, ISSN: 0022-2623, DOI: 10.1021/JM00031A014
- DOLEZAL ET AL: "Preparation, biological activity and endogenous occurrence of N<6>-benzyladenosines", BIOORGANIC & MEDICINAL CHEMISTRY, PERGAMON, GB, vol. 15, no. 11, 26 April 2007 (2007-04-26), pages 3737-3747, XP022047559, ISSN: 0968-0896, DOI: 10.1016/J.BMC.2007.03.038
- ARONOV A M ET AL: "Synthesis and structure-activity relationships of adenosine analogs as inhibitors of trypanosomal glyceraldehyde-3-phosphate dehydrogenase. Modifications at positions 5' and 8", BIOORGANIC & MEDICINAL CHEMISTRY LETTERS, PERGAMON, ELSEVIER SCIENCE, GB, vol. 8, no. 24, 15 December 1998 (1998-12-15), pages 3505-3510, XP004819382, ISSN: 0960-894X, DOI: 10.1016/S0960-894X(98)00635-0
- CLAUDIA HERFORTH ET AL: "Polymer-bound reagents for the introduction of spacer-modified biotin labels", BIOORGANIC & MEDICINAL CHEMISTRY, vol. 12, no. 11, 1 June 2004 (2004-06-01), pages 2895-2902, XP055054330, ISSN: 0968-0896, DOI: 10.1016/j.bmc.2004.03.038
- QING LIN ET AL: "Design of Allele-Specific Protein Methyltransferase Inhibitors", JOURNAL OF THE AMERICAN CHEMICAL SOCIETY, vol. 123, no. 47, 1 November 2001 (2001-11-01), pages 11608-11613, XP055054333, ISSN: 0002-7863, DOI: 10.1021/ja011423j
- GOLISADE A ET AL: "Anti-Malarial activity of N6-substituted adenosine derivatives. Part I", BIOORGANIC & MEDICINAL CHEMISTRY, PERGAMON, GB, vol. 10, 1 January 2002 (2002-01-01), pages 769-777, XP002430122, ISSN: 0968-0896, DOI: 10.1016/S0968-0896(01)00331-5
- LAUREN P SHEARMAN ET AL: "[125I]4-Aminobenzyl-5'-N-methylcarboxamid oadenosine ([125I]AB-MECA) labels multiple adenosine receptor subtypes in rat brain", BRAIN RESEARCH, vol. 745, no. 1-2, 1 January 1997 (1997-01-01), pages 10-20, XP055054441, ISSN: 0006-8993, DOI: 10.1016/S0006-8993(96)01120-1
- MAUBORGNE, ANNIE ET AL: "Adenosine receptor-mediated control of in vitro release of pain-related", EUROPEAN JOURNAL OF PHARMACOLOGY , 441(1-2), 47-55 CODEN: EJPHAZ; ISSN: 0014-2999, 2002, XP002692945,
- DE SARRO G ET AL: "EFFECTS OF ADENOSINE RECEPTOR AGONISTS AND ANTAGONISTS ON AUDIOGENIC SEIZURE-SENSIBLE DBA/2 MICE", EUROPEAN JOURNAL OF PHARMACOLOGY, ELSEVIER SCIENCE, NL, vol. 371, no. 2/03, 9 April 1999 (1999-04-09), pages 137-145, XP001004557, ISSN: 0014-2999, DOI: 10.1016/S0014-2999(99)00132-6
- SHEARDOWN, MALCOLM J. ET AL: "Unexpected neuroprotection observed with the adenosine", DRUG DEVELOPMENT RESEARCH , 39(1), 108-114 CODEN: DDREDK; ISSN: 0272-4391, 1996, XP002692946,
- NAI-KUEI HUANG ET AL: "Neuroprotective Principles from Gastrodia elata", JOURNAL OF NATURAL PRODUCTS, vol. 70, no. 4, 1 April 2007 (2007-04-01), pages 571-574, XP055054417, ISSN: 0163-3864, DOI: 10.1021/np0605182

## Description

### Technical Field

The present invention provides the identification, synthesis and use of compound active for treating neurodegenerative diseases, such as Huntington's disease. **Abbreviations:** A_{2A}R, A_{2A} adenosine receptor; Ac₂O, acetic anhydride; CGS, 6-amino-9-(5-ethylcarbamoyl-3,4-dihydroxy-oxolan-2-yl)-2 -{2-[4-(2-carboxyethyl)phenyl]ethylamino}purine; DIEA, diisopropylethylamine; DMF, *N,N*-dimethylformamide; DML, designed multiple ligands; ENT, equilibrative nucleotide transporter, ESI, eletrospray ionization; EtOAc, ethyl acetate; HD, Huntington's disease; hENT1, human equilibrative nucleoside transporter 1; HBA, hydrogen bond acceptor; HBD, hydrogen bond donor; HBTU. 2-(1*H*-benzotriazol-1-yl)-1,1,3,3-tetramethyluronium; HOBt, 1-hydroxybenzotriazole; HP, hydrophobic; HPLC, high-performance liquid chromatography; IR, infrared; JNK, *c*-Jun *N*-terminal kinase; MS, mass spectrometry; MsCl, methanesulfonyl chloride; MTT, 3-(4,5-dimethylthiazol-2-yl)-2,5-diphenyltetrazolium bromide; NBTI, *S*-(4-nitrobenzyl)-6-thioinosine; NMR, nuclear magnetic resonance; py, pyridine; PyBOP, benzotriazol-1-yl-oxytripyrrolidinophosphonium hexafluorophosphate; RA, ring aromatic; t-BuOH, tertiary butanol; TEMPO, 2,2,6,6-tetramethylpiperidinyl-1-oxy; THF, tetrahydrofuran; TLC, thin-layer chromatography; TsCl, *p*-toluenesulfonyl chloride; TsOH, *p*-toluenesulfonic acid; ZM, 4-(2-[7-Amino-2-{2-furyl}{1,2,4}triazolo{2,3-a}{1,3,5}tr iazin-5-yl-amino]ethyl)phenol.

### Background Art

### Introduction

Huntington's disease (HD) is an autosomal dominant neurodegenerative disease caused by a CAG trinucleotide expansion in the Huntingtin (Htt) gene, which shows chorea, dementia, and psychiatric symptoms. ¹⁻³ Effective treatment for HD has not yet been developed, though a few therapeutic agents with moderate effects have been reported.^{4, 5} It has been demonstrated that the selective A_{2A} adenosine receptor (A_{2A}R) agonist, CGS21680 (in short, CGS), can attenuate the HD symptoms in a transgenic mouse model,⁶ and this compound has been shown to be able to rescue the urea cycle deficiency of HD disease by enhancing the activity of the ubiquitin-proteasome system.⁷ However, CGS is known to exert strong immunosuppressive effect,⁸ among other side effects, and therefore is not suitable for clinical use. On the other hand, an adenosine analogue, designated as T1-11 (compound **1**), also an A_{2A}R agonist, is recently isolated from *Gastordia elata* and shown to prevent serum-derived PC12 cell apoptosis, suggesting its therapeutic potential in treating neural degenerative diseases.⁹

### Summary of the Invention

It has been recognized that the A_{2A}R and the adenosine transporter (such as the equilibrative nucleoside transporter ENT1) are both localized in the striatum,¹⁰⁻¹³ where the mutant Htt aggregate.¹⁴ Inhibition of the adenosine transporter would elevate the local concentrations of adenosine, and thereby increases the efficacy in agonizing the A_{2A}R. Interestingly, compound **1** is also found to be an ENT1 inhibitor. On the other hand, the potent immunosuppressant effect of CGS was a consequence of A_{2A}R signaling, which indicates that extremely strong binding affinity to A_{2A}R may not be a desirable property for clinically useful therapeutic agents.

It is therefore of considerable interest to modify compound **1** yet to retain its multiple-action property. Besides, transient or weakly binding compounds may be preferable than stably binding compounds,¹⁵ especially when targeting the A_{2A}R signaling system due to the aforementioned reasons. Designed multiple ligands (DML)^{16,17} are conceptually distinct from promiscuous compounds discovered by random screening, because they are rationally designed to optimize the desired properties. It has been proposed that DMLs may be advantageous for treatment of diseases with complex etiologies, e.g., asthma, obesity, cancer, and psychiatric diseases, compared to the individually-targeting compounds. ¹⁶⁻¹⁸ It has been conceived that the intrinsic redundancy and robustnesss of complex biological networks may be responsible to the failure of highly selective drugs to deliver the intended therapeutic effects.¹⁹ A recent assessment indicates that A_{2A}R pharmacology is indeed rather complex,²⁰ and therefore DMLs may be especially suitable for targeting the A_{2A}R signaling system. However, to design multiple-targeting ligands is often a challenging task,^{21,22} partly due to the difficulty in appropriate construction of the computational models for describing the interactions of the ligands with several targets, and partly due to the increased restraints of chemical synthesis, and satisfaction of physical chemical properties of compounds. Perhaps associated with the increasing complexity as the number of targets increases, currently most of reported DMLs are dual-function ligands.²²

We have described in our previously filed provisional application, U.S. Provisional Patent Application 61/260,932, a number of compounds that have been synthesized and that exhibit dual functions on both adenosine receptors and transporters. Other adenosine derivatives have been disclosed in WO2008/156513 or US2008/0176816 A1.

According to an aspect of the present invention, a composition is provided comprising an effective amount of a compound of formula: 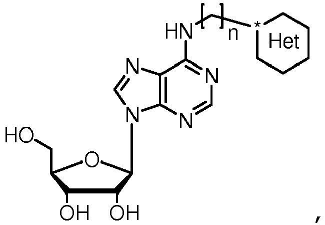 wherein n is 1 to 3, the (substituted)heterocycle contains 5- or 6-membered rings and the fused heterocycle contain nitrogen, oxygen or sulfur heteroatoms and the substituents are selected from the group consisting of hydrogen, fluorine, chlorine, bromine, iodine, hydroxyl C₁₋₆ alkyl, trifluoromethyl and (substituted phenyl, for use in treating neurodegenerative disease in a subject in need thereof.

In some embodiments, the compound is

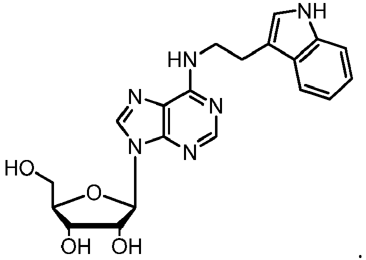

The neurodegenerative disease may be a protein-misfolding disease which is defined as a disease caused by protein-misfolding. In some embodiments, the neurodegenerative disease is selected from the group consisting of Alzheimer's disease, Parkinson's disease, amyotrophic lateral sclerosis, Prion disease, Huntington's disease, and spinal Cerebellar ataxias.

As another embodiment, the present invention provides for a composition comprising an effective amount of at least one of the above described compounds and a pharmaceutically acceptable carrier.

To rationally design the proper dual-action ligands as therapeutics for Huntington's disease, we first constructed two pharmacophore models, one for the A_{2A}R agonists, and the other for the ENT1 inhibitors. Three-dimensional pharmacophores are specific spatial distributions of chemical functional features of a series of compounds that target the same active site of a biomolecule and exert the same function,²³ which are particular useful if the high resolution X-ray or NMR structures of the target biomolecules are not yet available. Pharmacophore analysis has been successfully applied to numerous drug discovery tasks.²⁴⁻²⁹ Although the high resolution crystal structure of human adenosine A_{2A} receptor has been released recently,³⁰ it represents the conformation bound with the antagonist ZM241385 (in short, ZM), not with an agonist. On the other hand, the crystal structure of human ENT1 (hENT1) is not yet available, and there is also no suitable structural template for homology modeling.

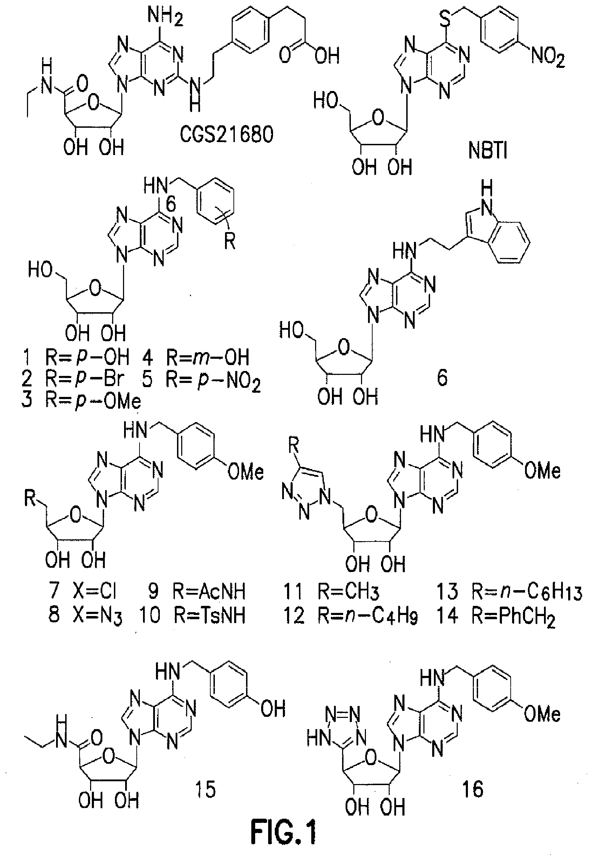

Based on the structural scaffold of 1, we set out to design a series of adenosine derivatives (Figure 1). Chemical modifications of adenosine were carried out if the pharmacophore fitting of the modified compound predicts acceptable activity. The competitive ligand binding assays were performed to verify if the designed compounds indeed bind to the A_{2A}R and ENT1 with good affinity. Finally, these compounds were assayed whether they could prevent apoptosis of the serum-deprived PC12 cells, which is a crucial indication for their potential for treating neurodegenerative diseases.^{31,32}

### Brief Description of the Drawings

**Figure 1****.** Structures of CGS, NBTI and some designed adenosine derivatives with modification at the *N*⁶- and *C*^{5'}-positions.
**Figure 2****.** The 3D pharmacophore model of the adenosine A_{2A} receptor agonists. (A) The geometric features of the pharmacophore model. Cyan: hydrophobic (HP), gold: ring aromatic (RA), magenta: hydrogen bond donor (HBD), green: hydrogen bond acceptor (HBA) (B) Fitting of CGS into the pharmacophore model. (C) Fitting of compound **1.** (D) Fitting of NBTI. (E) Fitting of compound **6.** (F) Fitting of compound **11.**
**Figure 3**. The 3D pharmacophore model of the human equilibrative nucleoside transporter (hENT1) inhibitors. (A) The geometric features of the pharmacophore model. Gold: ring aromatic (RA), green: hydrogen bond acceptor (HBA1: 3.431Å distnace from RA; HBA2: 10.388A distnace from RA). (B) Fitting of NBTI into the pharmacophore model. (C) Fitting of compound **1.** (D) Fitting of CGS. (E) Fitting of compound **6.** (F) Fitting of compound **11.**
**Figure 4****.** The scatter plot of the predicted p*K*ᵢ values of A_{2A}-R agonists versus the measured p*K*ᵢ values. The filled circles represent the training compounds, and the open circles the synthesized compounds.
**Figure 5**. The scatter plot of the predicted p*K*ᵢ values of ENT1 inhibitors versus the measured p*K*ᵢ values. The filled circles represent the training compounds, and the open circles the synthesized compounds.

### Description of the Embodiments

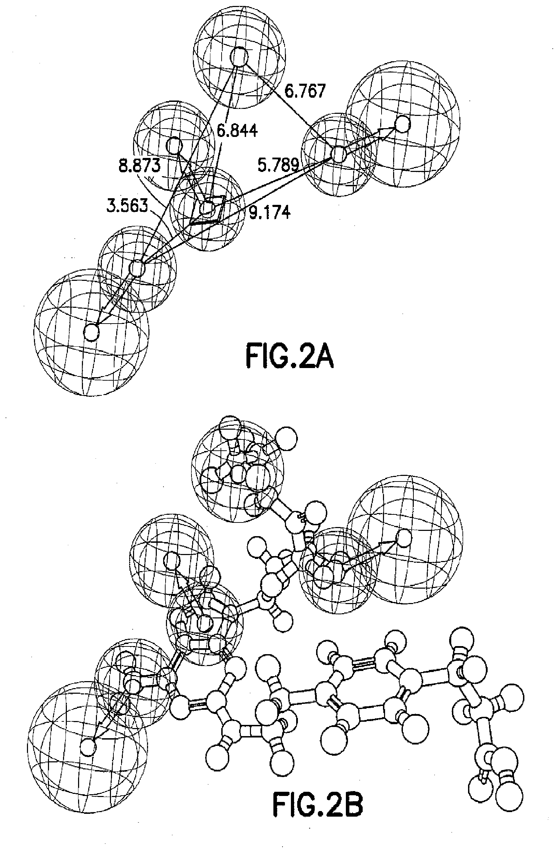

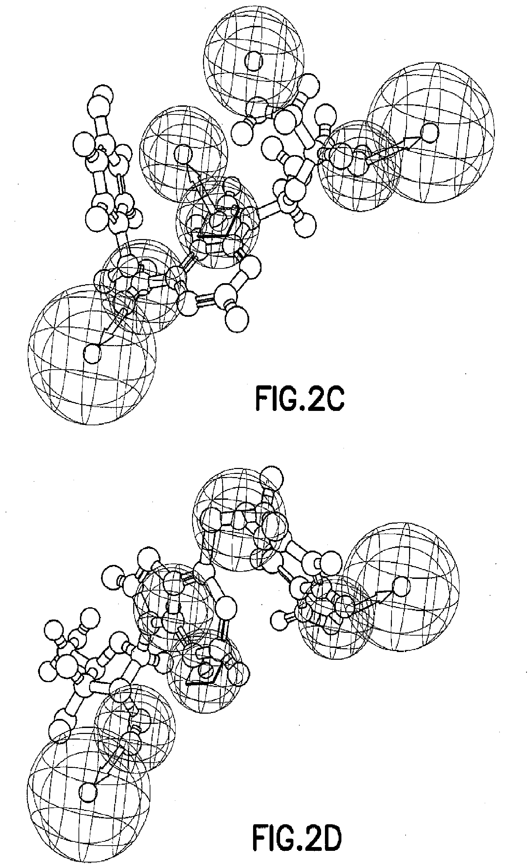

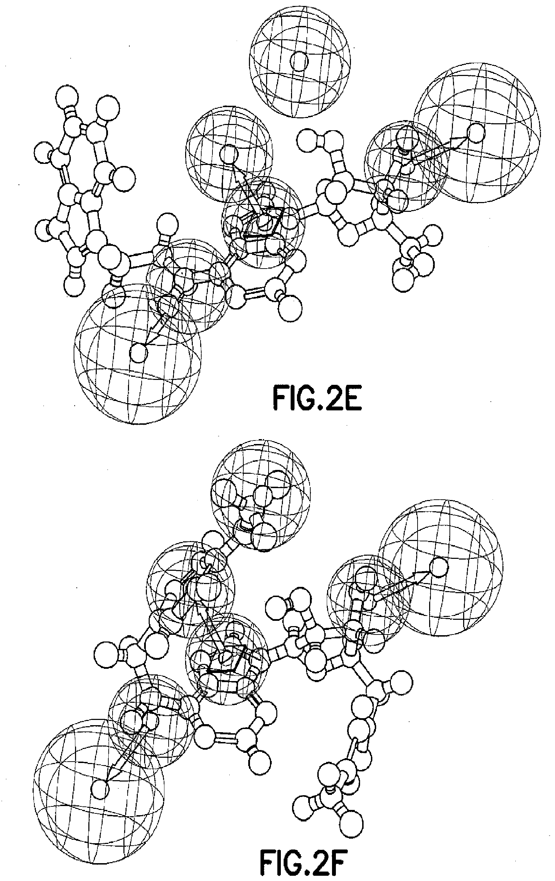

**Pharmacophore model of the human adenosine A_{2A} receptor agonists.** As part of the dual-pharmacophore drug design approach, a 3D-pharmacophore model of the human A_{2A}R (hA_{2A}R) agonists was first constructed to design the compounds that could function as hA_{2A}R agonists. The training set includes 25 compounds having large range of structural diversity and hA_{2A}R activity (*K*ᵢ from 1.2 nM to 187 µM) selected from the literature. A potent hA_{2A}R agonist CGS,³³ is also included in this training set. The HypoGen^{®} module of Catalyst^{®} of Accelrys^{® 34} was used to construct the pharmacophore model of these ligands. The constructed pharmacophore is illustrated in Figure 2A, which shows four geometric features including hydrophobic (HP, in cyan), ring aromatic (RA, in gold), hydrogen bond donor (HBD, in magenta) and hydrogen bond acceptor (HBA, in green) . For CGS all the four features of the constructed pharmacophore can be fitted nicely (Figure 2B). In contrast, S-(4-nitrobenzyl)-6-thioinosine (NBTI)³⁵ lacks a ring-aromatic fitting (Figure 2D), in agreement with its weak affinity as a hA_{2A}R ligand, though it exhibits a strong binding with adenosine transporter. The designed dual-action ligands **1, 6** and **11** fit at least three features in this pharmacophore model for A_{2A}R agonists (Figures 2C, 2E and 2F).

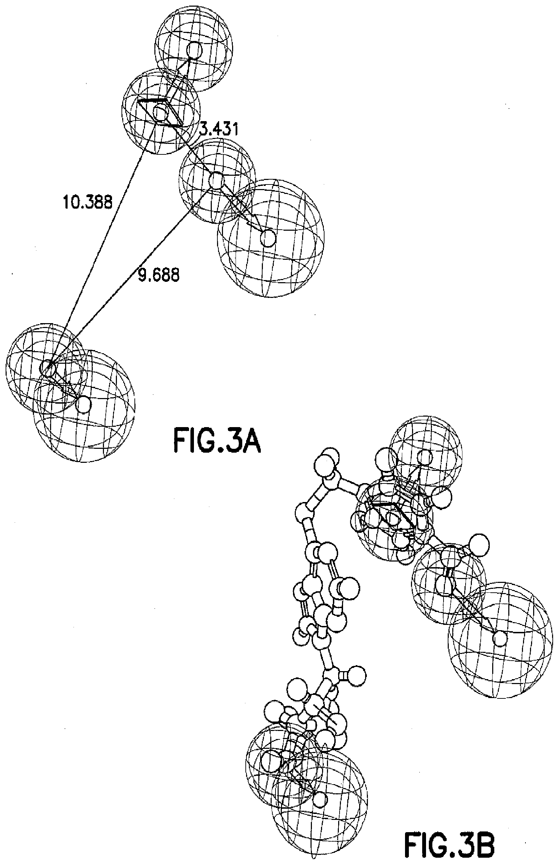

**Pharmacophore model of the equilibrative nucleoside transporter inhibitors.** To design dual-function compounds that act cooperatively as the hA_{2A}R agonists and the hENT1 inhibitors, the pharmacophore of hENT1 inhibitors was also constructed (Figure 3A). The training set includes 25 compounds possessing hENT1 inhibitory activity ranging from IC₅₀ of 0.29 nM to 32 µM, which were selected from the literature (see Supporting Information). The constructed pharmacophore model of the hENT1 inhibitors consists of only three features, including two hydrogen bond acceptors and one ring aromatic. All the five compounds (NBTI, **1**, CGS, **6,** and **11**) can fit into all these three features (Figures 3B-F). The different number of features between the pharmacophores of hA_{2A}R agonists and hENT1 inhibitors could be attributed to the nature of the training set compounds.

By carefully scrutinizing the structures in the hA_{2A}R agonists training set (see Supporting Information), we found that many of them possess a hydrophobic group in the 5' end of nucleoside, especially those compounds with higher potency, including CGS. Therefore, the constructed pharmacophore must include this important feature. On the contrary, in the hENT1 inhibitors training set, almost all the 5' end of the nucleoside possesses a polar hydroxyl group.

The pharmacophore differences among the two investigating targets also shed some light on the design of dual-function compounds. For example, compound **6** does not fit well to the hydrophobic feature in A_{2A} receptor pharmacophore model, and it is indeed less potent than CGS, which fits well to all features. However, compound **6** could fit well in all three features in hENT1 model.

As for compound **11,** it fits well to all features including hydrophobic of A_{2A}R pharmacophore mode. Nevertheless, it still fits well to hENT1 pharmacophore model, indicating the higher tolerance of ENT1 pharmacophore model. In summary, by comparing the features and compound-fitting qualities of these two pharmacophore models, we may hypothesize that the hA_{2A}R binding pocket has an important hydrophobic site, and the hENT1 binding pocket may be more flexible to accommodate the nucleosides with hydrophobic moieties at the 5' end in this series of compounds. Pharmacophore analyses provide us with an insight into the design and understanding of dual-function compound in the absence of the structural information of hENT1.

**Synthesis of adenosine derivatives.** A representative library of adenosine analogues (Figure 1) was developed based on the pharmacophore models. Compound **1,** originally isolated from *Gastrodia elata,*⁹ was synthesized in a high yield by the substitution reaction of 6-chloropurine ribofuranoside (**17**) with 4-hydroxybenzylamine (as the hydrochloric salt) in the presence of a base of diisopropylethylamine. As 4-hydroxybenzylamine was not commercially available, it was prepared in two steps from 4-hydroxybenzaldehyde via the condensation reaction with hydroxylamine to give an intermediate oxime, which was subsequently hydrogenated by the catalysis of Pd/C and HCl. By the procedures similar to that for compound **1,** a series of *N*⁶-substituted adenosine derivatives **2-6** were prepared by the substitution reactions of 6-chloropurine ribofuranoside with appropriate substituted benzylamines. In lieu of the conventional heating method, microwave irradiation was also applied to shorten the reaction time in the preparation of *N*⁶-(3-indolylethyl)adenosine (**6**).

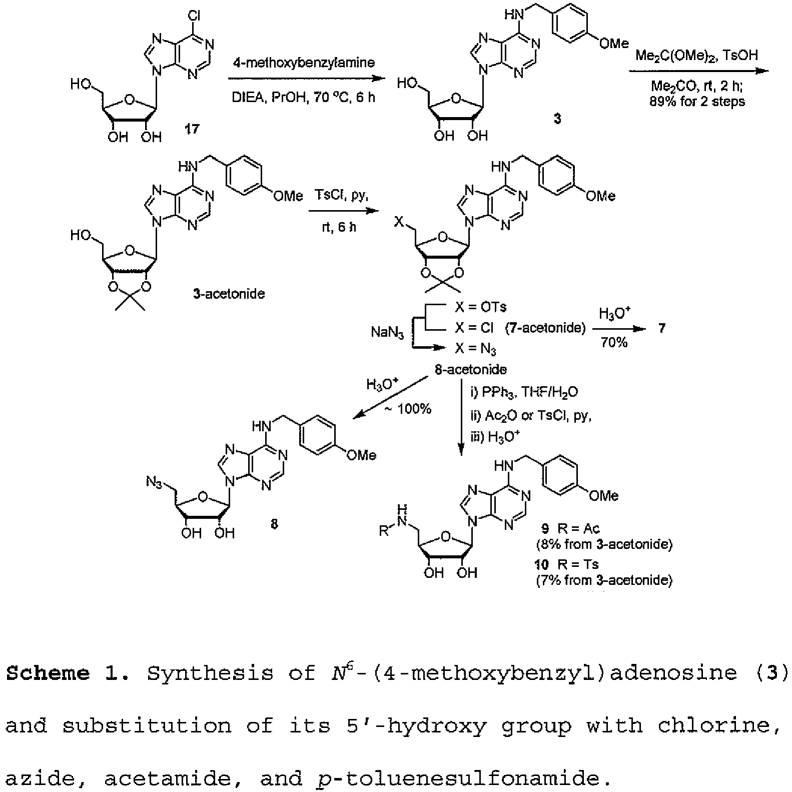

Treatment of *N*⁶-(4-methoxybenzyl)adenosine **3** with 2,2-dimethoxypropane in anhydrous acetone afforded the corresponding **3**-acetonide, which reacted with *p*-toluenesulfonyl chloride in the presence of pyridine to give a mixture of tosylate and chloride derivatives (Scheme 1). The tosylate derivative was unstable, whereas the chloride compound (**7**-acetonide) could be isolated by chromatography and subsequently hydrolyzed to give **7.** Without separation, this mixture was treated with sodium azide, followed by acid-catalyzed hydrolysis, to give an azido compound **8.** Alternatively, Staudinger reduction of **8**-acetonide gave an intermediate amine, which was converted to the corresponding acetamide **9** and sulfonamide **10** after removal of the 2',3'-isopropylidene group in acid.

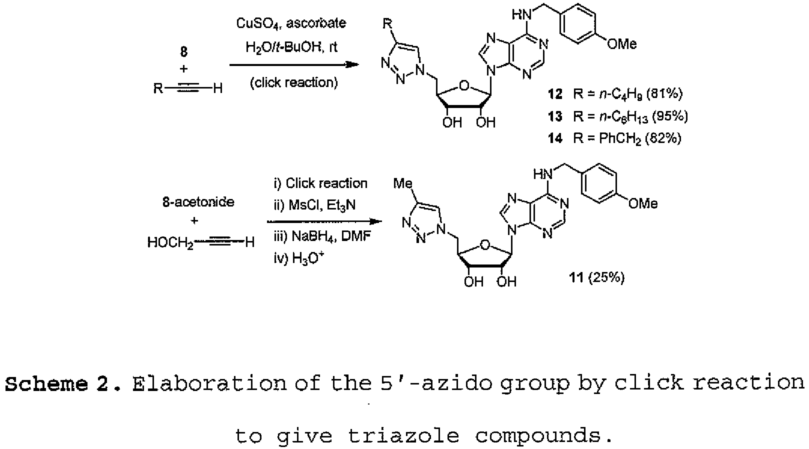

In another approach (Scheme 2), the Cu⁺-catalyzed 1,3-dipolar cycloaddition (click reaction)³⁸ of azido compound **8** with 1-hexyne, 1-octyne, and 3-phenyl-1-propyne afforded the triazole derivatives **12, 13** and **14,** respectively. Likewise, the click reaction of **8**-acetonide with propargyl alcohol gave a triazole compound, of which hydroxyl group was activated as a mesylate and then reduced by NaBH₄ to give a methyl group. Compound **11** was obtained after removal of the 2',3'-isopropylidene group.

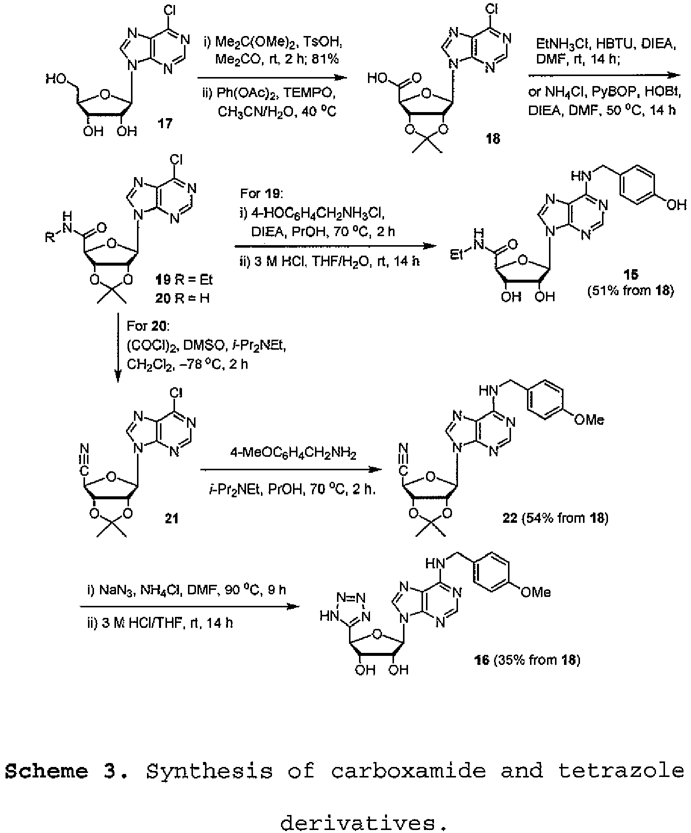

The acetonide of 6-chloropurine ribofuranoside was oxidized by (diacetoxyiodo) benzene with catalysis of 2,2,6,6-tetramethylpiperidinyl-1-oxy (TEMPO)³⁹ to give a carboxylic acid **18.** The coupling reactions of acid **18** with ethylamine and ammonia gave amides 19 and 20, respectively.

The chlorine atom in **19** was replaced by 4-hydroxybenzylamine, and the subsequent hydrolysis of the acetonide furnished amide **15.** On the other hand, amide **20** was converted to nitrile **21** on treatment with Me₂SO, oxalyl chloride and *i*-Pr₂NEt.⁴⁰ After the chlorine atom was substituted by 4-methoxybenzylamine, a 1,3-dipolar cycloaddition of the cyano group with NaN₃ introduced the desired tetrazole moiety at the C-5' position,41 giving **16** after removal of the 2',3'-isopropylidene group under acidic conditions.

**Biological evaluation of *N*⁶- and *C*^{5'}-modified adenosine derivatives.** The pharmacological properties of the prepared adenosine analogues were characterized by MDS Pharma Services using radioligand binding assays. The binding constants (*K*ᵢ) of some representative compounds are shown in Table 1. The potent A_{2A}R agonist CGS appears to lack the activity against ENT1, whereas the ENT1 inhibitor NBTI shows no binding ability with A_{2A}R. Neither CGS nor NBTI is dual-functional drug. Some prepared adenosine analogues exhibit the dual actions on A_{2A}R and ENT1, in particular, compounds **1, 4** and **6** showing the *K*ᵢ values in low micromolar and sub-micromolar range with A_{2A}R and ENT1, respectively. Except for **11** and **15,** the adenosine derivatives having modification at the *C*-5' position appeared to deteriorate their binding with A_{2A}R, though they still maintained high affinity with ENT1.

**Table 1. Binding activity of the N⁶- and C^{5'}-modified adenosine derivatives with adenosine receptor and transporter.^{a}**

| Compound | *K*ᵢ (µM) | |
|---|---|---|
| | A_{2A}-R*^{b}* | ENT1*^{c}* |
| CGS | 7.77 × 10⁻² | - |
| NBTI | > 10 | 2.9 × 10⁻⁴ |
| **1** | 2.62 | 5.38 × 10⁻¹ |
| **1**-acetonide | > 100 | > 100 |
| **2** | 14.4 | 1.44 × 10⁻² |
| **3** | 30.1 | 3.18 × 10⁻¹ |
| **4** | 3.21 | 3.72 |
| **5** | 27.7 | 6.51 × 10⁻³ |
| **6** | 4.39 | 3.47 |
| **7** | > 100 | 2.98 |
| **8** | - | 5.81 × 10⁻¹ |
| **9** | > 100 | 1.43 |
| **10** | > 100 | 2 × 10⁻¹ |
| **11** | 41.8 | 9.60 × 10⁻¹ |
| **12** | > 100 | 5.11 × 10⁻¹ |
| **13** | > 100 | 5.2 × 10⁻² |
| **14** | > 100 | 1.16 × 10⁻¹ |
| **15** | 20.3 | > 10 |
| **16** | > 100 | 1.17 |

| | | |
|---|---|---|
| *^{a}*The radioligand binding assays were performed by MDS Pharma Services Taiwan (Taipei, Taiwan) using standard binding protocols. *^{b}* Human adenosine A_{2A} receptor. *^{c}* Guinea pig equilibrium transporter 1. | | |

We have previously reported that compound **1** isolated from an aqueous methanolic extract of *Gastrodia elata* prevented apoptosis of serum-deprived PC12 cells by suppressing JNK activity.¹⁵ In this study, serum-deprived PC12 cells were treated with the compound at the indicated dose for 24 h. Cell viability was monitored by the MTT assay, and is expressed as a percentage of the MTT activity measured in the serum-containing group. At a concentration of 0.01 µM, compounds **4** and **6** also rescued PC12 cells from the apoptosis evoked by serum withdrawal equally as well as **1** according to the cell viability of MTT assays. Collectively, the dual function of these compounds in activation of adenosine receptor and in inhibition of adenosine transporter might synergistically increase the effective concentration of adenosine, especially when these two proteins are in proximity.

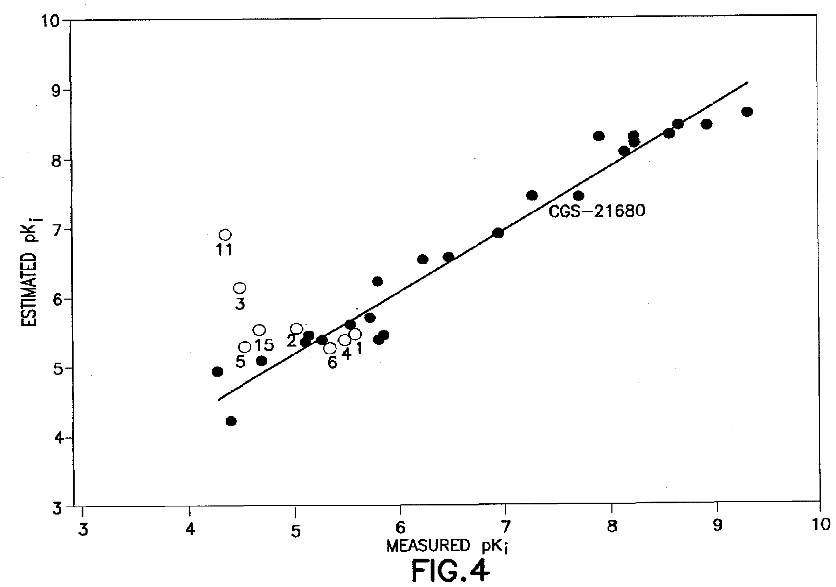

**Statistical assessment of pharmacophore models.** Figure 4 shows the scatter plot of the the experimental p*K*ᵢ versus predicted p*K*ᵢ values from the pharmacophore model of A_{2A}R agonists. The *r*² value of the predicted *K*ᵢ values versus the experimental *K*ᵢ values is 0.962, and the root-mean-square of error (rmse) is 0.658 kcal/mol. This pharmacophore model was further evaluated using the Fisher's randomization test for statistical significance, as implemented in the CatScramble module. The CatScrambler module scrambled the p*K*ᵢ values randomly for 19 times to generate new hypotheses (i.e., pharmacophore models) . None of the 19 hypotheses from the scrambled data had a cost lower than the reported hypothesis. Table 2 summarizes the fitted features of the compounds in Figure 4, along with the distance deviation of the fitted location of the feature on the compound from the center of the feature in the pharmacophore model. To reiterate, Table 2 is a quantitative representation of Figure 4. When a ligand is fitted into a pharmacophore, the quality of fitting (or mapping) is indicated by the "fit value." A higher fit value represents a better fit, and the computer fit values depends on two factors: the weights assigned to the pharmacophore features and how close the features in the molecules are to the exact locations of the features in the pharmacophore model.

**Table 2. Comparison of activities of compounds with the fitted number of features of the A_{2A}-R agonist pharmacophore model. The numbers are in the unit of Å.**

| Compound ID | HBD | HBA | RA | HP | Fit Value |
|---|---|---|---|---|---|
| CGS21680 | √ / 0.166 | √ / 0.125 | √ / 0.201 | √ / 0.436 | 10.6482 |
| **1** (T1-11) | √ / 0.121 | √ / 0.135 | √ / 0.248 | × | 8.68049 |
| NBTI | √ / 0.422 | √ / 0.304 | × | √ / 0.901 | 8.62848 |
| **6** | √ / 0.377 | √ / 0.359 | √ / 0.403 | × | 8.52907 |
| **11** | √ / 1.181 | √ / 0.445 | √ / 0.434 | √ / 0.544 | 9.59155 |

The potent A_{2A}R agonist CGS fits all the four features of the constructed pharmacophore, and the deviations of the fitted feature locations from the exact locations of the features of the pharmacophore model are also very small. Compared to CGS, compound 1 lacks a hydrophobic moiety to fit into the feature of the pharmacophore model, and therefore exhibited a reduced affinity. In contrast, NBTI lacks a ring-aromatic moiety, which completely abolishs the activity to agonize the adenosine A_{2A} receptor. This indicates that the ring-aromatic feature may be more important than the hydrophobic moiety in this pharmacophore model. Compound 6 does not fit the hydrophobic feature of the pharmacophore model, while compound **11** does show higher fit value. In contrast, the binding assays indicate that compound **6** (*K*ᵢ = 4.39 µM) exhibits ∼10-fold stronger binding affinity than compound **11** (*K*ᵢ = 41.8 µM). This can be rationalized by the fact that all the fitted locations of the features of the compound **6** have less deviations from the exact locations of the features.

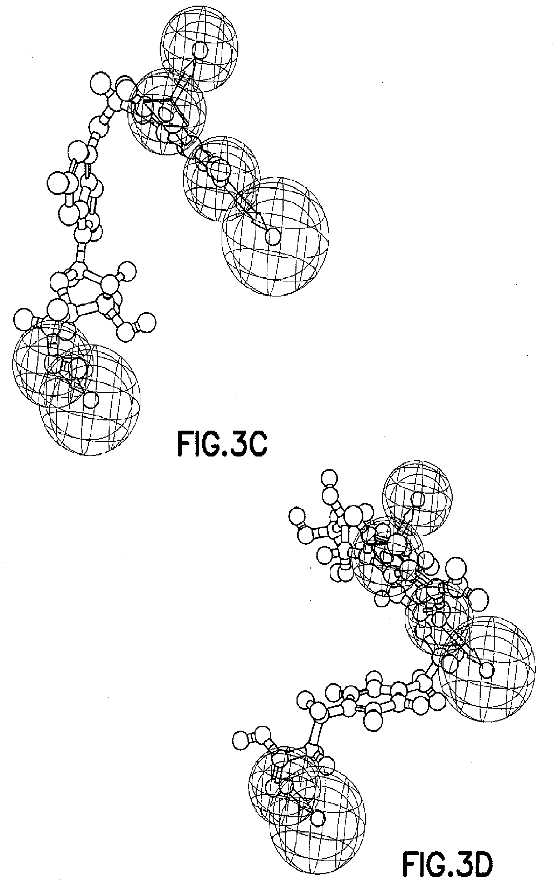

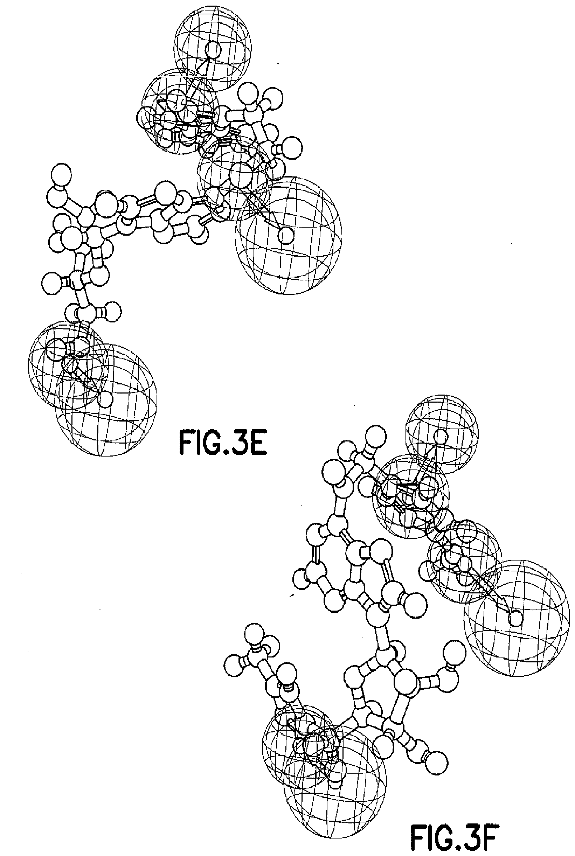

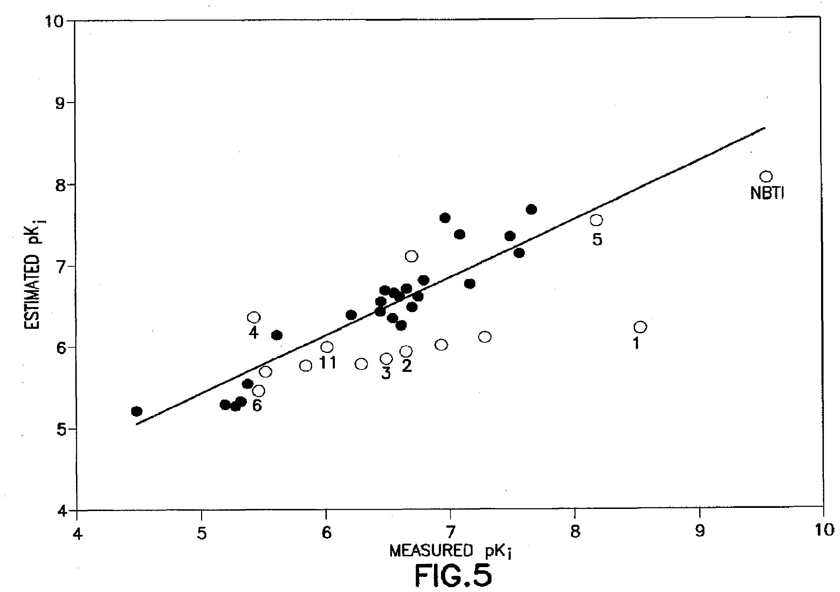

The constructed pharmacophore model of the hENT1 inhibitors consists of only three features, namely, a ring aromatic feature and two hydrogen bond acceptors. The *r*² value of the predicted *K*ᵢ values versus the experimental *K*ᵢ values is 0.927, and the rmse is 0.85 kcal/mol (Figure 5). This pharmacophore model was further evaluated by the CatScrambler module. All the five compounds (NBTI, **1,** CGS, **6,** and **11**) can fit into all these three features (Figures 3B-F), and therefore the deviations from the exact locations of the features need to be compared (Table 3). Apparently, the most potent inhibitor, NBTI, has the highest fit value and smallest deviation of all three features. The fit values of compounds **1, 11** and **6** are 6.61, 6.4 and 5.86, respectively, which are consistent with the ranking of their measured activity. CGS (with a high fit value 7.1) is obviously an outlier of this model, since this compound has no inhibitory activity toward hENT1. However, CGS is the only compound with a ring aromatic feature fitted on to the nucleoside moiety (Figure 3) . It is thus important to carefully examine whether the fitted functional group is indeed the same as the functional groups of the training set compounds that define the consensus feature in the pharmacophore analysis. The "fit value" alone can not be considered the measure of fitness.

**Table 3. Comparison of activities of compounds with the fitted number of features of the ENT1 inhibitor pharmacophore model. The numbers are in the unit of Å.**

| Compound ID | HBD | HBA | RA | Fit Value |
|---|---|---|---|---|
| NBTI | √ / 0.421 | √ / 0.647 | √ / 0.793 | 5.92748 |
| **1** | √ / 0.544 | √ / 0.619 | √ / 0.586 | 4.94133 |
| CGS21680 | √ / 0.567 | √ / 1.276 | √ / 0.358 | 5.37611 |
| **6** | √ / 0.421 | √ / 0.647 | √ / 0.493 | 5.40138 |
| **11** | √ / 0.74 | √ / 0.588 | √ / 0.942 | 4.35311 |

### Conclusion

We have adopted a dual-pharmacophore modeling approach to design dual-action compounds targeting the A_{2A}R signaling system. Based on the structural scaffold of 1, we designed and synthesized a series of adenosine derivatives and carried out chemical modifications of adenosine if the pharmacophore fitting of the modified compound predicts acceptable activity. The competitive ligand binding assays verified that the designed compounds indeed bind to both A_{2A}R and ENT1 with moderate affinity. The effective amount of the designed compounds for therapeutic treatment of neurodegenerative diseases, including Huntington's disease, is 1.5-2.5 mg/kg, based on oral dosage of the representatibe T1-11 in mice. The preferred route of administration of the designed compounds is oral administration, either in immediate release or slow release forms. Finally, these compounds were shown to prevent apoptosis of the serum-deprived PC12 cells, which is a crucial indication for their potential for treating neurodegenerative diseases.

### Experimental Section

**Materials and Methods.** All reagents and solvents were of reagents grade and were used without further purification unless otherwise specified. Tetrahydrofuran and diethyl ether were distilled from Na/benzophenone and CH₂Cl₂ was distilled from CaH₂. All air or moisture sensitive experiments were performed under argon. All glasses were dried in oven for more than 2 hours and used after cooling to room temperature in desiccators. Microwave reactions were conducted using a focused single mode microwave unit (CEM Discover). The machine consists of a continuous focused microwave power delivery system with operator selectable power output.

Melting points were recorded on a Yanaco micro apparatus. Optical rotations were measured on digital polarimeter of Japan JASCO Co. DIP-1000. [α]_{D} values are given in units of 10⁻¹deg cm² g⁻¹. Infrared (IR) spectra were recorded on Nicolet Magna 550-II. NMR spectra were obtained on Varian Unity Plus-400 (400 MHz) and chemical shifts (δ) were recorded in parts per million (ppm) relative to δ_{H} 7.24/ δ_{C} 77.0 (central line of t) for CHCl_{3/}CDCl₃, δ_{H} 2.05/ δ_{C} 29.92 for (CH₃)₂CO/(CD₃)₂CO, δ_{H} 3.31/ δ_{C} 49.0 for CH₃OH/CD₃OD, and δ_{H} 2.49 (m) /. δ_{C} 39.5 (m) for (CH₃)₂SO/(CD₃)₂SO. The splitting patterns are reported as s (singlet), d (doublet), t (triplet), q (quartet), m (multiplet) and br (broad). Coupling constants (J) are given in Hz. The ESI-MS experiments were conducted on a Bruker Daltonics BioTOF III high-resolution mass spectrometer. Analytical thin-layer chromatography (TLC) was performed on E. Merck silica gel 60 F₂₅₄ plates (0.25 mm). Compounds were visualized by UV, anisaldehyde or ninhydrin spray. Column chromatography was carried out on columns packed with 70-230 mesh silica gel. Purity of compounds tested on A_{2A}-R and ENT1 was assessed to be ≥95% by HPLC (Agilent HP-1100) with detection at 280 nm wavelength.

**Construction of pharmacophore models.** The HypoGen module of Catalyst^{®} of Accelrys was used to construct the pharmacophore model of human A_{2A}R agonists and human ENT 1 inhibitors. The chemical structures of the training set compounds and their binding affinities to the human A_{2A}R (or human ENT 1) were collected from the literature (see Supporting Information) .⁴¹⁻⁴⁴ It is important to ensure that the activity of the training compounds cover at least four orders of magnitude, with at least three compounds in each log scale. ^{23,45} It is also recommended to select compounds with larger chemical diversity as the training set.⁴⁵ Each compound was sketched with ChemDraw, and then imported into Catalyst 4.11. The "Best" option was used for conformation generation. In Catalyst^{®}, the Poling algorithm was used to generate 250 conformations, whose energies are less than 20.0 kcal/mol from the lowest energy of all the conformations. Five molecular features were selected, namely, hydrophobic (HPh), hydrogen bond acceptor (HBA), hydrogen bond donor (HBD), positively ionizable atom (PI), and negatively ionizable atom (NI). All these compounds were loaded into the Catalyst's spreadsheet and the default uncertainty of 3 were assigned. All other parameters are as default.

***N*⁶-(4-Hydroxybenzyl)adenosine (1).⁹** Hydroxylamine hydrochloride (1.29 g, 18.6 mmol) and sodium acetate (1.67 g, 20.4 mmol) were added to a solution of 4-hydroxybenzaldehyde (1.25 g, 10.2 mmol) in ethanol (20 mL). The reaction mixture was stirred at room temperature for 6 h. Ethanol was removed under reduced pressure. The residue was added water, and then extracted with Et₂O (3 ×). The combined organic layer was dried over MgSO₄. After the volatiles were removed by rotary evaporation under reduced pressure, the residue was recrystallized from CH₂Cl₂ to give the oxime of 4-hydroxybenzaldehyde (1.3 g, 93%). C₇H₇NO₂; light yellow solid, mp 92.0-93.6 °C.

A solution of the above-prepared oxime (342 mg, 2.5 mmol) and concentrated hydrochloric acid (1 mL) in ethanol (20 mL) was subjected to hydrogenation at atmospheric pressure in the presence of 10% Pd/C (80 mg) for 4 h. The reaction mixture was filtered through Celite. The filtrate was concentrated to yield the hydrochloric salt of 4-hydroxybenzylamine as light yellow solids, which were used for the next step without further purification.

A mixture of 4-hydroxybenzylamine (395 mg, as the hydrochloric salt), 6-chloropurine riboside (143 mg, 0.5 mmol), and diisopropylethylamine (DIEA, 2 mL, 12 mmol) in 1-propanol (25 mL) was heated to 70 °C for 6 h. The mixture was concentrated under reduced pressure, and triturated with water to give white precipitates, which were filtered to yield the title compound **1** (151 mg, 81%). The purity of product was > 99% as shown by HPLC on an Inertsil ODS-3 column (4.6 × 250 mm, 5 µm) with elution of 0.1% TFA/MeOH (6:4). C₁₇H₁₉N₅O₅; white powder, mp 208.7-209.2 °C (lit.⁹ mp 216-219 °C) ; [α]²⁰_{D} = -64.5 (DMSO, *c* = 1) (lit.⁹ [α]²¹_{D} = -87 (MeOH, *c* = 0.1)); TLC (MeOH/EtOAc (1:9)) *R_{f}* = 0.3; ¹H NMR (DMSO-*d*₆, 400 MHz) δ 9.22 (1 H, s), 8.34 (1H, s), 8.30 (1 H, br s), 8.18 (1 H, s), 7.12 (2 H, d, *J* = 8.0 Hz), 6.65 (2 H, d, *J* = 8.0 Hz), 5.86 (1 H, d, *J* = 5.6 Hz), 5.41 (2 H, m), 5.18 (1 H, d, *J* = 5.6 Hz), 4.60 (2 H, m), 4.13 (1 H, q, *J* = 4.6, 7.4 Hz), 3.95 (1 H, q, *J* = 3.4, 6.2 Hz), 3.66 (1 H, m), 3.53 (1 H, m); ¹³C NMR (DMSO-*d*₆, 400 MHz) δ 155.3, 153.6, 151.6, 147.6, 139.1, 129.5, 127.9 (2 ×), 119.2, 114.4 (2 ×), 87.6, 85.6, 73.3, 70.5, 61.5, 42.4; ESI-HRMS calcd for C₁₇H₂₀N₅O₅: 374.1459, found: *m*/*z* 374.1412 [M + H]⁺.

***N*⁶-(4-Bromobenzyl)adenosine (2).** By a procedure similar to that for compound **1,** 6-chloropurine riboside (98 mg, 0.34 mmol) was treated with 4-bromobenzylamine hydrochloride (5 equiv) and diisopropylethylamine (2 mL, 12 mmol) in 1-propanol (20 mL) at 70 °C for 7 h to give the title compound **2** (41 mg, 28%) after recrystallization from MeOH. The purity of product was 95% as shown by HPLC on an HC-C18 column (Agilent, 4.6 × 250 mm, 5 µm) with elution of gradients of 30-60% aqueous CH₃CN in 20 min. C₁₇H₁₈BrN₅O₄; white powder; mp 179.2-179.4 °C; [α]²⁶_{D} = -50.1 (CH₃OH, *c* = 1.0); TLC (MeOH/EtOAc (1:9)) *R_{f}*= 0.69; IR *v*ₘₐₓ (neat) 3333, 2925, 2865, 2358, 1621, 1486, 1408, 1336, 1298, 1224, 1121, 1081, 1011 cm⁻¹; ¹H NMR (DMSO-*d*₆, 400 MHz) δ 8.47 (1 H, br s), 8.37 (1 H, s), 8.19 (1 H, s), 7.47 (2 H, d, *J* = 8.0 Hz), 7.28 (2 H, d, *J* = 8.0 Hz), 5.88 (1 H, d, *J* = 6.4 Hz), 5.43 (1 H, d, *J* = 6.0 Hz), 5.37 (1 H, dd, *J* = 2.4, 7.2 Hz), 5.18 (1 H, d, *J* = 4.8 Hz), 4.66 (2 H, br s), 4.61 (1 H, dd, *J* = 5.6, 6.0 Hz), 4.08-4.16 (2 H, m) 3.96 (1 H, d, *J* = 2.8 Hz), 3.64-3.69 (2 H, m), 3.57-3.52, (2 H, m); ¹³C NMR (DMSO-*d*₆, 100 MHz) δ 154.2, 152.1, 148.3, 139.9, 139.3, 130.9 (2 ×), 130.6 (2 ×), 119.6, 87.9, 85.9, 73.5, 70.7, 61.7, 42.4; ESI-HRMS calcd for C₁₇H₁₈BrN₅O₄: 436.0620, found: *m*/*z* 436.0654 [M + H]⁺. ***N*⁶-(4-Methoxybenzyl)adenosine (3).** By a procedure similar to that for **1,** 6-chloropurine riboside (100 mg, 0.35 mmol) was treated with 4-methoxybenzylamine (243 mg, 1.77 mmol) and diisopropylethylamine (2 mL, 12 mmol) in 1-propanol (20 mL) at 70 °C for 7 h to give the title compound **3** (95 mg, 76%) after recrystallization from MeOH. The purity of product was > 99% as shown by HPLC on an HC-C18 column (Agilent, 4.6 × 250 mm, 5 µm) with elution of gradients of 30-60% aqueous CH₃CN in 20 min. C₁₆H₁₈N₆O₄; white powder; mp 147.6-148.2 °C; [α]²⁰_{D} = -55.3 (DMSO, *c* = 1.0) (lit.²¹ [α]²⁵_{D} = -66.15 (MeOH, *c* = 0.12)); TLC (MeOH/EtOAc (1:9)) *R_{f}* = 0.60; ¹H NMR (DMSO-*d*₆, 400 MHz) δ 8.38 (2 H, m), 8.19 (1 H, br s), 8.30 (1 H, br s), 7.25 (2 H, d, *J* = 8.4 Hz), 6.83 (2 H, d, *J* = 8.4 Hz), 5.87 (1 H, d, *J* = 6.0 Hz), 5.45-5.38 (2 H, m), 5.19 (1 H, d, *J* = 3.2 Hz), 4.61 (2 H, s), 4.14 (1 H, s), 3.95 (1 H, dd, *J* = 3.2, 6.8 Hz), 3.70-3.64 (4 H, m), 3.57-3.52 (1 H, m); ¹³C NMR (DMSO-*d*₆, 400 MHz) δ 157.8, 154.2, 152.1, 148.2, 139.7, 131.7, 128.3 (2 ×), 119.6, 113.4 (2 ×), 87.8, 85.8, 73.4, 70.6, 61.6, 55.0; ESI-MS calcd for C₁₆H₁₉N₆O₄: 388.1615, found: *m*/*z* 388.1620 [M+H]⁺.

***N*⁶-(3-Hydroxybenzyl)adenosine (4).** By a procedure similar to that for **1,** 6-chloropurine riboside (143 mg, 0.5 mmol) was treated with 3-hydroxybenzylamine hydrochloric salt (395 mg, 2.46 mmol) and diisopropylethylamine (2 mL, 12 mmol) in 1-propanol (25 mL) at 70 °C for 6 h. The mixture was concentrated under reduced pressure, and triturated with water to give white precipitates, which were filtered to yield the title compound 4 (151 mg, 81%). The purity of product was >99% as shown by HPLC on an HC-C18 column (Agilent, 4.6 × 250 mm, 5 µm) with elution of gradients of 30-60% aqueous CH₃CN in 20 min. C₁₇H₁₉N₅O₅; white powder, mp 195.6-196.5 °C; TLC (MeOH/EtOAc (1:9)) *R_{f}* = 0.3; [α]²⁰_{D} = -46.5 (DMSO, *c* = 1.0); ¹H NMR (DMSO-*d*₆, 400 MHz) δ 9.22 (1 H, s), 8.34 (1H, s), 8.30 (1 H, br s), 8.18 (1 H, s), 7.12 (2 H, d, *J* = 8.0 Hz), 6.65 (2 H, d, *J* = 8.0 Hz), 5.86 (1 H, d, *J* = 5.6 Hz), 5.41 (2 H, m), 5.18 (1 H, d, *J* = 5.6 Hz), 4.60 (2 H, m), 4.13 (1 H, q, *J* = 4.6, 7.4 Hz), 3.95 (1 H, q, *J* = 3.4, 6.2 Hz), 3.66 (1 H, m), 3.53 (1 H, m); ¹³C NMR (DMSO-*d*₆, 400 MHz) δ 155.3, 153.6, 151.6, 147.6, 139.1, 129.5, 127.9 (2 ×), 119.2, 114.4 (2 ×), 87.6, 85.6, 73.3, 70.5, 61.5, 42.4; ESI-HRMS calcd for C₁₇H₂₀N₅O₅: 374.1459, found: *m*/*z* 374.1412 [M+H]⁺.

***N*⁶-(4-Nitrobenzyl)adenosine (5).** By a procedure similar to that for **1,** 6-chloropurine riboside (102 mg, 0.36 mmol) was treated with 4-nitrobenzylamine hydrochloride (336 mg, 1.78 mmol) and diisopropylethylamine (2 mL, 12 mmol) in 1-propanol (20 mL) at 70 °C for 7 h to give the title compound **5** (55.6 mg, 38%) after recrystallization from MeOH. The purity of product was > 99% as shown by HPLC on an HC-C18 column (Agilent, 4.6 × 250 mm, 5 µm) with elution of gradients of 30-60% aqueous CH₃CN in 20 min. C₁₇H₁₈N₆O₆; white crystals from MeOH; mp 167.8-168.6 °C (lit.⁴ mp 174-176 °C); [α]²⁰_{D} = -50.7 (DMSO, *c* = 0.5); TLC (MeOH/EtOAc (1:9)) *R_{f}*= 0.45; ¹H NMR (DMSO-*d*₆, 400 MHz) δ 8.62 (1 H, br s), 8.40 (1 H, s), 8.18-8.14 (3 H, m), 7.56 (2 H, d, *J* = 8.4 Hz), 5.88 (1 H, d, *J* = 6.0 Hz), 5.44 (1 H, d, *J* = 5.6 Hz), 5.34 (1 H, t, *J* = 5.6 Hz) 7.12 (2 H, *d, J=* 8.0 Hz); ¹³C NMR (DMSO-*d*₆, 400 MHz) δ 152.1, 147.9, 146.1, 140.0, 127.8 (2 ×), 123.3 (2 ×), 87.8, 85.8, 73.4, 70.6, 61.6, 42.7; ESI-HRMS calcd for C₁₇H₁₉N₆O₆: 403.1361, found: *m*/*z* 403.1372 [M+H]⁺.

**N⁶-**(**3**-**Indolylethyl**) **adenosine (6) .** In a round-bottomed flask (10 mL) were placed a solution of 6-chloropurine ribonucleoside (71 mg, 0.25 mmol), trypamine (101 mg, 0.63 mmol) and diisopropylethylamine (0.24 mL, 2.88 mmol) in EtOH (3 mL). The flask was placed into the cavity of a focused monomode microwave reactor, and irradiated at 150 W for 10 min in refluxing EtOH. The mixture was concentrated by rotary evaporation, and the residue was purified by flash chromatography (silica gel; MeOH/EtOAc (1:9)) to give the title compound **6** (85 mg, 83%). The purity of product was > 99% as shown by HPLC on an HC-C18 column (Agilent, 4.6 × 250 mm, 5 µm) with elution of gradients of 30-60% aqueous CH₃CN. C₂₀H₂₂N₆O₄; white powder; mp 187.0-187.2 °C; [α]²⁰_{D} = -55.7 (CH₃OH, *c* = 1.0); TLC (MeOH/EtOAc (1:9)) *R_{f}* = 0.41; ¹H NMR (DMSO-*d*₆, 400 MHz) δ 10.78 (1 H, s), 8.33 (1 H, s), 8.25 (1 H, s), 7.96 (1 H, br s), 7.61 (1 H, d, *J* = 7.2 Hz), 7.32 (1 H, d, *J* = 9.2 Hz), 7.18 (1 H, s), 7.05 (1 H, t, *J* = 8.0 Hz), 5.80 (1 H, d, *J* = 6.0 Hz), 5.47-5.44 (2 H, m), 5.20 (1 H, d, *J* = 4.4 Hz), 4.61 (1 H, d, *J* = 5.6 Hz), 4.14 (1 H, d, *J* = 2.8 Hz), 3.96 (1 H, d, *J* = 3.2 Hz), 3.77 (1 H, br s), 3.69-3.52 (2 H, m), 3.01 (2 H, t, *J* = 7.2 Hz) ; ¹³C NMR (DMSO-*d*₆, 100 MHz) δ 154.3, 152.2, 148.0, 139.5, 136.0, 127.1, 122.4, 120.8, 119.6, 118.3, 118.1, 111.7, 111.2, 87.9, 85.8, 73.4, 70.6, 61.6, 40.5, 25.1; ESI-HRMS calcd for C₂₀H₂₃N₆O₄ : 411.1775, found: *m*/*z* 411.1750 [M + H]⁺.

**5**'-**Chloro**-**5**'-**deoxy**-***N*⁶**-(**4**-**methoxybenzyl**)**adenosine (7).** The reaction of **3**-acetonide (2.96 g, 6.9 mmol) with *p*-toluenesulfonyl chloride (6.3 g, 34.6 mmol) in pyridine (6.0 mL) gave a mixture of sulfonate and the chloride derivatives (5:1) as shown by the ¹H NMR spectrum. The mixture was separated by flash chromatography (silica gel; C_{H2}Cₗ₂/MeOH (100:1)). The sulfonate compound showed ¹H NMR (CDCₗ₃, 400 MHz) δ 8.25 (1 H, s), 7.66 (1 H, s), 7.60 (2 H, d, *J* = 8.0 Hz), 7.29 (2 H, d, *J* = 8.4 Hz), 7.14 (2 H, d, *J* = 8.0 Hz), 6.86 (2 H, d, *J* = 8.4 Hz), 6.00 (1 H, s), 5.31 (1 H, d, *J* = 6.0 Hz), 5.04 (1 H, dd, *J* = 3.2, 6.0 Hz), 4.78 (br, s), 4.43-4.47 (1 H, m), 4.20-4.30 (2 H, m), 3.78 (3 H, s), 2.36 (3 H, s), 1.58 (3 H, s), 1.36 (3 H, s). The sulfonate compound was unstable, and further conversion was not pursued.

The chloride compound (7-acetonide) showed ¹H NMR (CDCl₃, 400 MHz) δ 8.3 (1 H, s), 7.74 (1 H, s), 7.29 (2 H, d, *J* = 8.4 Hz), 6.86 (2 H, d, *J* = 8.4 Hz), 6.07 (1 H, s), 5.45 (1 H, d, *J* = 6.4 Hz), 5.16 (1 H, s), 4.78 (br, s), 4.50 (1 H, d, *J=* 6.0 Hz), 3.83-3.77 (4 H, m), 3.63-3.59 (1 H, m), 1.62 (3 H, s), 1.40 (3 H, s). Compound 7-acetonide (8.5 mg, 0.019 mmol) was dissolved in 3 M HCl/THF (1:1, 130 µL). The mixture was stirred at room temperature for 14 h, and neutralized with saturated NaHCO₃ aqueous solution. The mixture was concentrated under reduced pressure, and the residue was extracted with CH₂Cl₂ and H₂O. The organic layer was dried over MgSO₄, filtered, and concentrated to give the title compound 7 (5.9 mg, 70%). The purity of product was > 99% as shown by HPLC on an HC-C 18 column (Agilent, 4.6 × 250 mm, 5 µm) with elution of gradients of 30-60% aqueous CH₃CN. C₁₈H₂₀ClN₅O₄; white solid; mp 182.0-182.6 °C; TLC (EtOAc) *R_{f}*= 0.4; [α]²¹_{D} = -45.4 (THF, *c* = 0.68); IR_{*v*max} (neat) 3329, 3127, 1630, 1586, 1515, 1487, 1421, 1379, 1354, 1297, 1253, 1174, 1111, 1063, 1031 c^{m-1}; ¹H NMR (CD₃OD, 400 MHz) δ 8.26 (1 H, s), 8.23 (1H, s), 7.30 (2 H, d, *J* = 8.8 Hz), 6.86 (2 H, d, *J* = 8.8 Hz), 6.03 (1 H, d, *J* = 4.8 Hz), 4.77 (1 H, t, *J* = 5.2 Hz), 4.72 (2 H, br s), 4.38 (1 H, t, *J* = 5.2 Hz), 4.27 (1 H, t, *J* = 4.8 Hz), 3.94 (1 H, dd, J*=*6.8, 4.8 Hz), 3.83 (1 H, dd, *J* =6.8, 4.8 Hz), 3.76 (1H, s); ¹³C NMR (DMSO-*d*₆, 100 MHz) δ 157.9, 154.2,152.5,148.6, 139.6,131.8,128.4 (2 ×), 119.4,113.5 (2 ×), 87.5, 83.7, 72.7, 71.3, 55.1, 44.9, 42.4; ESI-HRMS calcd for C₁₈H₂₁ClN₅O₄: 406.1282, found: *m*/*z* 406.1279 [M + H]⁺.

**5'-Azido-5'-deoxy-*N*⁶-(4-methoxybenzyl)adenosine (8).** The azido compound **8**-acetonide (41.5 mg, 0.092 mmol) was stirred in 3 M HCl/THF (1:1, 0.26 mL) at room temperature for 14 h, and then neutralized with saturated NaHC_{O3} aqueous solution. The mixture was concentrated under reduced pressure, and the residue was extracted with C_{H2}C₁₂ and _{H2}O. The organic layer was dried over MgS_{O4}, filtered, and concentrated to give a quantitative yield of the title compound 8. The purity of product was 98% as shown by HPLC on an HC-C18 column (Agilent, 4.6 × 250 mm, 5 µm) with elution of gradients of 30-60% aqueous C_{H3}CN. _{C18H20N8O4}; white solid; mp 190.6-191.1 °C; TLC (C_{H2}Cₗ₂/MeOH (9:1)) *_{Rf}*= 0.5; [α^{]2}_{4D} = -13.9 (CHCₗ₃, *c* = 1.0); IR _{*v*max} (neat) 3333, 2925, 2853, 2102, 1620, 1512, 1465, 1337, 1295, 1246, 1176, 1110, 1069, 1034 c^{m-1}; ¹H NMR (C_{D3}OD, 400 MHz) δ 8.27 (1 H, br s), 8.24 (1 H, br s), 7.30 (2 H, d, *J* = 8.4 Hz), 6.86 (2 H, d, *J* = 8.4 Hz), 6.02 (1 H, d, *J* = 4.8 Hz), 4.77 (1 H, t, *J* = 5.2 Hz), 4.73 (2 H, br s), 4.36 (1 H, t, *J* = 5.2 Hz), 4.18 (1 H, q, *J* = 4.0 Hz), 3.65 (2 H, t, *J* = 3.6 Hz); ¹³C NMR (DMSO-_{*d*6}, 100MHz) 157.9, 154.2, 152.4, 148.7, 139.7, 131.8 ,128.4 (2 ×), 119.5, 113.5 (2 ×), 87.8, 72.8, 70.9, 55.1, 51.8, 42.4 ; ESI-HRMS calcd for _{C18H20N8O4}: 413.1686, found: *m*/*z* 413.1686 [^{M}+ H^{]+}.

**5'-Azido-5'-deoxy-2',3'-*O*-isopropylidene-*N*⁶-(4-methoxybe nzyl)adenosine (8-actonide).** To the acetonide of *N*⁶-(4-methoxybenzyl)adenosine (**3**-acetonide, 2.96 g, 6.9 mmol) in anhydrous pyridine (36 mL) was added a solution of *p*-toluenesulfonyl chloride (6.3 g, 34.6 mmol) in anhydrous pyridine (6.0 mL) dropwise via syringe to a solution of . The mixture was stirred at room temperature for 6 h. Pyridine was removed under reduced pressure, and the residue was extracted with CH₂Cl₂ and H₂O. The organic layer was dried over MgSO₄, filtered, and concentrated to give a mixture of sulfonate and the chloride derivatives (5:1) as shown by the ¹H NMR spectrum.

The above-prepared mixture was dissolved in anhydrous DMF (70 mL), and sodium azide (1.34 g, 20.6 mmol) was added. The mixture was stirred at 80 °C for 6 h, and then concentrated under reduced pressure. The residue was extracted with CH₂Cl₂ and H₂O. The organic layer was dried over MgSO₄, filtered, and concentrated to give a pale yellow oil, which was purified by flash chromatography (silica gel; CH₂Cl₂/meOH (100:1)) to give **8**-acetonide (653 mg, 21% overall yield). C₂₁H₂₄N₈O₄; colorless oil; TLC (EtOAc/Hexane (6:4)) *R_{f}* = 0.39; [α]²³_{D} = +5.0 (EtOAc, *c* = 1.0) ; IR *v*ₘₐₓ (neat) 3280, 2987, 2931, 2101, 1618, 1512, 1478, 1375, 1330, 1296, 1248, 1211, 1154, 1091 cm⁻¹; ¹H NMR (CDCl₃, 400 MHz) δ 8.38 (1 H, br s), 7.72 (1H, br s), 7.26 (2 H, d, *J* = 8.8 Hz), 6.84 (2 H, d, *J* = 8.8 Hz), 6.37 (1H, br s), 6.06 δ (1 H, d, *J* = 2.0 Hz), 5.46 -5.44 (1 H, m), 5.07 -5.05 (1 H, m), 4.77 (2 H, br s), 4.38 -4.35 (1 H, m), 3.77 (3 H, s), 3.51-3.62 (2 H, m), 1.61 (3 H, s), 1.39 (3 H, s) ; ¹³C NMR (CDCl₃, 100 MHz) δ 158.7, 154.5, 153.1, 147.9, 139.0, 130.2, 128 .8 (2 ×), 120.1, 114.4, 113.8 (2 ×), 90.4, 85.6, 83.9, 82.0, 55.2, 52.2, 43.7, 29.7, 27.1, 25.3; ESI-HRMS calcd for C₂₁H₂₄N₈O₄ : 453.1999, found: *m*/*z* 453.1999 [M + H]⁺.

**5'-Acetamido-5'-deoxy-*N*⁶-(4-methoxybenzyl)adenosine (9).** The azido compound **8**-acetonide (95 mg, 0.21 mmol) was stirred with triphenylphosphine (66 mg, 0.24 mmol) in THF/H₂O (10:1, 2 mL) at room temperature for 4.5 h. The mixture was concentrated under reduced pressure. The residue was taken up with CH₂Cl₂ and H₂O, and acidified with HCl solution (1 M) until pH = 2. The aqueous phase was separated, neutralized with saturated NaHCO₃ aqueous solution, and extracted with CH₂Cl₂. The organic extract was dried over MgSO₄, filtered, and concentrated to yield a crude amine product.

The crude amine was treated with acetic anhydride (98.6 µL, 1.05 mmol) in anhydrous pyridine (0.2 mL). The mixture was stirred at room temperature for 1.5 h, and then concentrated under reduced pressure. The residue was extracted with CH₂Cl₂ and H₂O. The organic layer was dried over MgSO₄, filtered, concentrated, and purified by flash chromatography (silica gel; CH₂Cl₂/MeOH (98:2)) to give the acetonide of compound **9** (56 mg, 57% yield for 2 steps) . The purity of product was > 99% as shown by HPLC on an HC-C18 column (Agilent, 4.6 × 250 mm, 5 µm) with elution of gradients of 30-60% aqueous CH₃CN. C₂₃H₂₈N₆O₅; colorless oil; TLC (CH₂Cl₂/MeOH (98:2)) *R_{f}* = 0.2; [α]²⁵_{D} = -146.6 (CHCl₃, *c* = 1.0); IR *v*ₘₐₓ (neat) 3280, 3062, 2989, 2930, 2835, 2358, 1667, 1620, 1513, 1376, 1336, 1296, 1246, 1215, 1096, 1034 cm⁻¹; ¹H NMR (CDCl₃, 400 MHz) δ 8.36-8.38 (2 H, m), 7.73 (1 H, s), 7.28 (2 H, d, *J* = 8.8 Hz), 6.86 (2 H, d, *J* = 8.8 Hz), 6.15 (1 H, br s), 5.77 (1 H, d, *J* = 4.8 Hz), 5.26 (1 H, t, *J* = 4.8 Hz), 4.81 (1 H, dd, *J* = 4.0, 2.4 Hz), 4.76 (2 H, br s), 4.47-4.48 (1 H, m), 4.11-4.17 (1 H, m), 3.79 (3 H, s), 3.24 (1 H, d, *J* = 14.4 Hz), 2.15 (3H, s), 1.61 (3 H, s), 1.34 (3 H, s); ¹³C NMR (CDCl₃, 100 MHz) δ 170.5, 158.8, 154.8, 152.7, 147.7, 139.7, 130.0 ,128.9 (2 ×), 121.1, 114.6, 113.9 (2 ×), 92.5, 83.3, 82.2, 81.3, 55.3, 43.9, 41.1, 27.6, 25.4, 23.2; ESI-HRMS calcd. for C₂₃H₂₈N₆O₅: 469.2190, found *m*/*z* 469.2193 [M + H]⁺.

The acetonide of **9** (17.2 mg, 0.037 mmol) was stirred in 3 M HCl/THF (1:1, 0.1 mL) at room temperature for 14 h, and then neutralized with saturated NaHCO₃ aqueous solution. The mixture was concentrated under reduced pressure, and the residue was extracted with CH₂Cl₂ and H₂O. The organic layer was dried over MgSO₄, filtered, and concentrated to give the title compound **9** (11 mg, 70%). The purity of product **9** was 99% as shown by HPLC on an HC-C18 column (Agilent, 4.6 × 250 mm, 5 µm) with elution of gradients of 30-60% aqueous CH₃CN in 20 min. C₂₀H₂₄N₆O₅; white powder; mp 121.1-121.6 °C; TLC (CH₂Cl₂/MeOH (9:1)) *R_{f}* = 0.5; [α]²⁵_{D} = -108.7 (THF, *c* = 0.89) ; IR *v*ₘₐₓ (neat) 3275, 3071, 2923, 2852, 2360, 1621, 1512, 1375, 1339, 7.297, 1245, 1175, 1126, 1076 cm⁻¹; ¹H NMR (CDCl₃, 400 MHz) δ 8.76 (1 H, s), 8.27 (1H, s), 7.24 (2 H, d, *J* = 8.4 Hz), 6.81 (2 H, d, *J* = 8.4 Hz), 6.54 (1 H, s), 5.70 (1 H, d, *J* = 5.6 Hz), 4.72 (3 H, d, *J* = 5.6 Hz), 4.23 (1 H, s), 4.18 (1 H, s), 3.98-4.03 (1 H, m), 3.75 (3 H, s), 3.13 (1 H, d, *J* = 14.0 Hz), 2.02 (3 H, s); ¹³C NMR (DMSO, 1.00 MHz) δ 169.4, 157.9, 154.3, 152.3, 148.3, 140.2, 131.8, 128.4 (2 ×), 119.8, 113.5 (2 ×), 87.9, 83.6, 72.6, 71.3, 55.1, 42.4, 41.1, 22.7; ESI-HRMS calcd for C₂₀H₂₄N₆O₅: 427.1730, found: *m*/*z* 427.1727 [M + H]⁺.

**5**'-**(*p***-**Tolylsulfonamido**)-**5**'-**deoxy**-***N*⁶**-(**4**-**methoxybenzyl**)**adenosine (10).** The crude amine compound (∼72.4 mg), prepared by Staudinger reduction of **8**-acetonide, was dissolved in anhydrous pyridine (1.1 mL), and a solution of *p*-toluenesulfonyl chloride (159.4 mg, 0.87 mmol) in anhydrous pyridine (1.12 mL) was added dropwise via syringe. The mixture was stirred at room temperature for 4.5 h. Pyridine was removed under reduced pressure, and the residue was extracted with C_{H2}Cₗ₂ and _{H2}O. The organic layer was dried over MgS_{O4}, filtered, and concentrated to give a colorless oil, which was purified by flash chromatography (silica gel; C_{H2}Cₗ₂/MeOH (120:1 to 100:1)) to give the acetonide of compound 10 (40.4 mg, 41% yield). _{C28H32N606}; TLC (EtOAc/Hexane (10:1)) *_{Rf}*= 0.25; [α^{]2}_{7D} = +2.7 (CHCₗ₃, *c* = 1.0); IR _{*v*max} (neat) 2925, 2862, 2360, 1621, 1513, 1480, 1383, 1329, 1301, 1247, 1215, 1158, 1087, 1034 c^{m-1}; ¹H NMR (CDCₗ₃, 400 MHz) δ 9.29 (1 H, d, *J* = 10.0 Hz), 8.60 (1 H, s), 7.71 (2 H, d, *J* = 8.0 Hz), 7.63 (1 H, s), 7.28 (2 H, d, *J* = 8.4 Hz), 7.24 (2 H, d, *J* = 8.0 Hz), 6.85 (2 H, d, *J* = 8.4 Hz), 6.35 (1 H, br s), 5.70 (1 H, d, *J* = 4.8 Hz), 5.24 (1 H, t, *J* = 5.6 Hz), 4.93 (1 H, d, *J* = 6.0 Hz), 4.78 (2 H, br s), 4.49 (1 H, d, *J* = 1.2 Hz), 3.79 (3 H, s), 3.43-3.47 (1 H, m), 3.13 (1 H, dd, *J* = 2.4, 10.8 Hz), 2.37 (3 H, s), 1.59 (3 H, s), 1.31 (3 H, s); ¹³C NMR (CDCₗ₃, 100 MHz) δ 158.9, 154.8, 153.1, 142.8, 139.2, 137.2, 130.0, 129.6 (2 ×), 129.0(2 ×), 126.7 (2 ×), 114.5, 114.0 (2 ×), 93.4, 82.8, 82.3, 82.6, 55.3, 45.3, 44.1, 27.6, 25.2, 21.5; ESI-HRMS calcd for _{C28H32N6O6}S: 581.2182, found: *m*/*z* 581.2198 [M + H^{]+}.

The acetonide of **10** (41.7 mg, 0.072 mmol) was stirred in 3 M HCl/THF (1:1,0.2 mL) at room temperature for 14 h, and then neutralized with saturated NaHCO₃ aqueous solution. The mixture was concentrated under reduced pressure, and the residue was extracted with CH₂Cl₂ and H₂O. The organic layer was dried over MgSO₄, filtered, and concentrated to give the crude product, which was purified by flash chromatography (silica gel; CH₂Cl₂/MeOH = 25:1) to give the title compound 10 (68%). The purity of product was > 99% as shown by HPLC on an HC-C 18 column (Agilent, 4.6 × 250 mm, 5 µm) with elution of gradients of 30-60% aqueous CH₃CN. C₂₅H₂₇N₆O₆S; TLC (EtOAc/Hexane (10:1)) *R_{f}* = 0.25; [α]²⁶_{D} = +25.9 (THF, *c* = 0.8); IR *v*ₘₐₓ (neat) 3356, 2926, 2860, 2362, 1620, 1513, 1461, 1330, 1301, 1247, 1159, 1069, 1038 cm⁻¹; ¹H NMR (CD₃OD, 400 MHz) δ 7.91 (1 H, s), 7.66 (1H, s), 7.27 (2 H, d, *J* = 8.0 Hz), 6.84-6.87 (4 H, m), 6.41 (2 H, d, *J* = 8.4 Hz), 5.40 (1 H, d, *J* = 6.4 Hz), 4.28 (3 H, br s), 3.77-3.82 (3 H, m), 2.88-2.89 (1 H, m), 2.85 (1 H, d, *J=* 2.8 Hz), 2.71 (1 H, d, *J* = 2.8 Hz), 2.68 (1 H, d, *J* = 3.2 Hz), 1.92 (3 H, s); ¹³C NMR (CD₃OD, 100 MHz) δ 160.6, 156.3, 153.9, 149.3, 144.9, 142.0, 138.7, 132.2, 130.9 (2 ×), 130.1 (2 ×), 128.0 (2 ×), 121.7, 115.1 (2 ×), 91.6, 85.9, 74.4, 73.1, 55.9, 46.2, 44.8, 21.6; ESI-HRMS calcd for C₂₅H₂₈N₆O₆S: 541.1869, found: *m*/*z* 541.1877 [M + H]⁺.

**5**'-**Deoxy**-**5**'-(**4**-**methyl**-**1,2**,**3**-**triazol**-1-**yl**)-***N*⁶**-(**4**-**methoxyb enzyl)adenosine (11).** A mixture of azido compound **8**-acetonide (313 mg, 0.69 mmol), CuSO₄●5H₂O (24.9 mg), sodium ascorbate (61.4 mg) and propargyl alcohol in H₂O/*t*-BuOH (1:1, 7 mL) was stirred at room temperature for 12 h, and then concentrated under reduced pressure. The residue was extracted with CH₂Cl₂ and H₂O. The organic layer was dried over MgSO₄, filtered, and concentrated to yield a triazole acetonide (∼ 220 mg) as colorless oil. TLC (CH₂Cl₂/MeOH (9:1)) *R_{f}* = 0.5; ESI-HRMS calcd for C₂₄H₂₈N₈O₅: 509.2261, found: *m*/*z* 509.2267 [M + H]⁺.

The above-prepared triazole compound was stirred with triethylamine (0.15 mL, 1.08 mmol) and methylsulfonyl chloride (0.08 mL, 1.08 mmol) in anhydrous CH₂Cl₂ (4.3 mL) at room temperature for 2 h. The mixture was concentrated under reduced pressure, and the residue was extracted with CH₂Cl₂ and H₂O. The organic layer was dried over MgSO₄, filtered, and concentrated to yield a mesylate compound as colorless oil. TLC (EtOAc/Hex (4:1)) *R_{f}* = 0.45; ESI-HRMS calcd for C₂₅H₃₀N₈O₇SNa: 609.1856, found: *m*/*z* 609.1876 [M + Na]⁺.

The mesylate was treated with NaBH₄ (24.5 mg, 0.65 mmol) at 0 °C in DMF, and then heated to 60 °C for 6 h. The mixture was concentrated under reduced pressure, and the residue was extracted with CH₂Cl₂ and H₂O. The organic layer was dried over MgSO₄, filtered, and concentrated to give **11**-acetonide as colorless oil. TLC (EtOAc/Hex (4:1)) *R_{f}* = 0.25; ESI-HRMS calcd for C₂₄H₂₈N₈O₄: 493.2312, found: *m*/*z* 493.2312 [M + H]⁺. The acetonide of **11** was stirred in 3 M HCl/THF (1:1, 0.33 mL) at room temperature for 14 h, and then neutralized with saturated NaHCO₃ aqueous solution. The mixture was concentrated under reduced pressure, and the residue was dissolved in THF, filtered, and concentrated to give the title compound **11** (48.1 mg, 25% overall yield). The purity of product was 98% as shown by HPLC on an HC-C18 column (Agilent, 4.6 × 250 mm, 5 µm) with elution of gradients of 30-60% aqueous CH₃CN. C₂₁H₂₄N₈O₄; white powder; mp 183.0-183.2 °C; TLC (CH₂Cl₂/MeOH (9:1)) *R_{f}* = 0.12; [α]²⁷_{D} = +20.3 (CH₃OH, *c* = 0.45); IR *v*ₘₐₓ (neat) 3217, 2921, 2850, 2685, 1620, 1513, 1470, 1337, 1297, 1244, 1176, 1111, 1058 cm⁻¹; ¹H NMR (CD₃OD, 400 MHz) δ 8.22 (1 H, s), 7.99 (1H, s), 7.45 (1 H, s), 7.31 (2 H, d, *J* = 8.8 Hz), 6.87 (2 H, d, *J* = 8.8 Hz), 5.96 (1 H, d, *J* = 4.0 Hz), 4.82-4.68 (5 H, m), 4.46 (1 H, t, *J* = 4.0 Hz), 4.34 (1 H, q, *J* = 4.0 Hz), 3.77 (3 H, s), 2.15 (3 H, s); ¹³C NMR (CDCl₃, 100 MHz) δ 158.7, 154.2, 152.9, 148.0, 142.8, 138.8, 130.1, 128.8 (2 ×), 123.1, 119.6, 113.8 (2 ×), 89.3, 82.2, 73.4, 70.8, 55.2, 50.9, 43.9, 10.5; ESI-HRMS (negative mode) calcd for C₂₁H₂₄N₈O₄: 451.1842, found: *m*/*z* 451.1843 [M - H]⁻.

**5'-(4-Butyl-1,2,3-triazol-1-yl)-5'-deoxy-*N*⁶-(4-methoxybenzyl)adenosine (12).** A mixture of azido compound **8** (13.31 mg, 0.032 mmol), CuSO₄•SH₂O (0.40 mg), sodium ascorbate (0.95 mg) and 1-hexyne in H₂O/*t*-BuOH (1:1, 430 µL) was stirred at room temperature for 30 h, and then concentrated under reduced pressure. The residue was extracted with CH₂Cl₂ and H₂O. The organic layer was dried over MgSO₄, filtered, and concentrated to give the title compound **12** (12.82 mg, 81%). The purity of product was > 95% as shown by HPLC on an HC-C18 column (Agilent, 4.6 × 250 mm, 5 µm) with elution of gradients of 30-60% aqueous CH₃CN. C₂₄H₃₀N₈O₄; white powder; mp 105.0-105.4 °C; TLC (CH₂Cl₂/MeOH (9:1)) *R_{f}=* 0.38; [α]²³_{D} = -12.2 (CHCl₃, *c* = 1.0); IR *ν*ₘₐₓ (neat) 3333, 3140, 2929, 2858, 2360, 1619, 1512, 1464, 1333, 1297, 1245, 1176, 1094, 1037 cm⁻¹; ¹H NMR (CDCl₃, 400 MHz) δ 8.28 (1 H, s), 7.73 (1H, s), 7.27 (2 H, d, *J* = 8.4 Hz), 7.15 (1 H, s), 6.84 (2 H, d, *J* = 8.4 Hz), 6.13 (1 H, br s), 5.89 (1 H, d, *J* = 5.2 Hz), 4.73 (3 H, dd, *J* = 9.6, 4.8 Hz), 4.61-4.67 (2 H, m), 4.51 (1 H, d, *J* = 4.4 Hz), 4.34 (1 H, br s), 3.78 (3 H, s), 2.45-2.57 (2 H, m), 1.39-1.46 (2 H, m), 1.19-1.28 (2 H, m), 0.84 (3 H, t, *J* = 7.6 Hz); ¹³C NMR (CDCl₃, 100 MHz) δ 158.8, 154.3, 152.9, 148.5, 148.0, 138.8, 130.1 , 128.9 (2 ×), 122.5, 119.7, 113.9 (2 ×), 89.5, 82.5, 73.5, 70.9, 55.3, 51.1, 44.0, 31.2, 25.0, 22.3, 13.8; ESI-HRMS calcd for C₂₄H₃₀N₈O₄: 493.2312, found: *m*/*z* 493.2316 [M + H]⁻.

**5'-Deoxy-5'-(4-hexyl-1,2,3-triazol-1-yl)-*N*⁶-(4-methoxybenzyl)adenosine (13).** A mixture of azido compound **8** (10.30 mg, 0.025 mmol), CuSO₄•5H₂O (0.31 mg), sodium ascorbate (0.73 mg) and 1-octyne (4.46 µL, 0.03 mmol) in H₂O/*t*-BuOH (1:1, 333 µL) was stirred at room temperature for 37 h, and then concentrated under reduced pressure. The residue was extracted with CH₂Cl₂ and H₂O. The organic layer was dried over MgSO₄, filtered, and concentrated to give the title compound **13** (12.36 mg, 94.6%). The purity of product was > 98% as shown by HPLC on an HC-C18 column (Agilent, 4.6 × 250 mm, 5 µm) with elution of gradients of 30-60% aqueous CH₃CN. C₂₆H₃₄N₈O₄; white powder; mp 101.4-102.1 °C; TLC (CH₂Cl₂/MeOH (9:1)) *R_{f}*= 0.33; [α]²⁵_{D} =-9.7 (CHCl₃, *c* = 1.0); IR *ν*ₘₐₓ (neat) 3332, 3148, 2926, 2855, 2359, 1620, 1513, 1463, 1336, 1297, 1246, 1175, 1109, 1037 cm⁻¹; ¹H NMR (CDCl₃, 400 MHz) δ 8.26 (1 H, s), 7.67 (1H, s), 7.26 (2 H, d, *J* = 8.4 Hz), 7.16 (1 H, s), 6.82 (2 H, d, *J* = 8.4 Hz), 6.31 (1 H, br s), 5.91 (1 H, d, *J* = 4.4 Hz), 4.63-4.75 (4 H, m), 4.58 (1 H, t, *J =* 4.8 Hz), 4.44-4.51 (2 H, m), 3.76 (3 H, s), 2.42-2.56 (2 H, m), 1.20-1.24 (8 H, m), 0.82 (3 H, t, *J* = 6.8 Hz); ¹³C NMR (DMSO-*d*₆, 100 MHz) δ 157.9, 154.2, 152.5, 148.4, 146.7, 139.7, 131.8, 128.4 (2 ×), 122.5, 119.5, 113.6 (2 ×), 93.9, 87.9, 82.4, 72.7, 70.9, 55.1, 51.2, 42.4, 31.1, 28.9, 28.3, 25.0, 22.2, 14.1; ESI-HRMS calcd for C₂₆H₃₄N₈O₄: 521.2625, found: m/z 521.2628 [M + H]⁻.

**5'-(4-Benzyl-1,2,3-triazol-1-yl)-5'-deoxy-*N*⁶-(4-methoxybenzyl)adenosine (14).** A mixture of azido compound **8** (11.97 mg, 0.029 mmol), CuSO₄•5H₂O (0.36 mg), sodium ascorbate (0.85 mg) and 3-phenyl-1-propyne in H_{2O}/*t*-BuOH (1:1, 386 µL) was stirred at room temperature for 30 h, and then concentrated under reduced pressure. The residue was extracted with CH₂Cl₂ and H₂O The organic layer was dried over MgSO₄ filtered, and concentrated to give the title compound **14** (12.57 mg, 82%). The purity of product was 99% as shown by HPLC on an HC-C18 column (Agilent, 4.6 × 250 mm, 5 µm) with elution of gradients of 30-60% aqueous CH₃CN. C₂₇H₂₈N₈O₄; white powder, mp 123.3-123.9 °C; TLC (CH₂Cl₂/MeOH (9:1)) *R_{f}* = 0.38; [α]²⁶_{D} = +9.3 (CHCl₃, *c* = 1.0); IR *ν*ₘₐₓ (neat) 3329, 3143, 3029, 2925, 2835, 2359, 1620, 1479,1434, 1333, 1296, 1245, 1176, 1109, 1034 cm⁻¹; ¹HNMR (CDCl₃, 400 MHz) δ 8.27 (1 H, s), 7.66 (1H, s), 7.27 (2 H, d, *J* = 8.8 Hz), 7.10-7.19 (3 H, m), 7.03-7.06 (3 H, m), 6.81 (2 H, d, *J* = 8.8 Hz), 6.20 (1 H, br s), 5.86 (1 H, d, *J* = 4.8 Hz), 4.75 (2 H, br s), 4.67 (1 H, dd, *J* = 10.0, 4.8 Hz), 4.56-4.60 (2 H, m), 4.36 (1 H, t, *J* = 4.4 Hz), 3.86 (2 H, dd, *J* = 18.4, 16.0 Hz), 3.75 (3 H, s); ¹³C NMR (CDCl₃, 100 MHz) 158.8, 154.2, 152.9, 147.1, 138.8, 138.2 , 130.1, 128.9 (2 x), 128.4 (2 ×), 128.3 (2 ×), 126.4, 123.3, 119.7, 113.9 (2 ×), 89.4, 82.3, 73.6, 70.9, 55.3, 51.1, 44.0, 31.8; ESI-HRMS calcd for C₂₇H₂₈N₈O₄: 527.2155, found: *m*/*z* 527.2140 [M + H]⁻.

**3,4-Dihydroxy-5-[6-(4-hydroxybenzylamino)-purin-9-yl]-te trahydrofuran-2-carboxylic acid ethylamide (15).** The acetonide derived from 6-chloropurine ribofuranoside (**17**-acetonide, 158 mg, 0.48 mmol) was stirred with PhI(OAc)₂ (509 mg, 1.56 mmol) and 2,2,6,6-tetramethylpiperidinyl-1-oxy (TEMPO, 15.4 mg, 0.1 mmol) in a degassed CH₃CN/H₂O solution (1:1, 2.6 mL) at 40 °C for 4 h. The mixture was concentrated under reduced pressure to yield a crude acid product **18.**

The crude acid was treated with ethylamine (117 mg, as the hydrochloric salt), *O*-(benzotriazol-1-yl)-*N*,*N*,*N*',*N*'-tetramethyluronium hexafluorophosphate (HBTU, 375 mg, 0.72 mmol) and diisopropylethylamine (0.5 ml, 2.89 mmol) in anhydrous DMF (11.6 mL) at room temperature for 14 h. The mixture was concentrated under reduced pressure. The residue was extracted with CH₂Cl₂ and H₂O. The organic layer was dried over MgSO₄, filtered, concentrated, and purified by flash chromatography (silica gel; EtOAc/hexane (1:1)) to yield an amide product **19** as colorless oil.

The amide product was treated with 4-hydroxybenzylamine (385 mg, 2.4 mmol as the hydrochloric salt) and diisopropylethylamine (2.8 mL), 16.9 mmol) in 1-propanol (28 mL) at 70 °C for 2 h. The mixture was concentrated under reduced pressure, and the residue was extracted with CH₂Cl₂ and H₂O The organic layer was dried over MgSO₄, filtered, concentrated, and purified by flash chromatography (silica gel; CH₂Cl₂/MeOH (97:3)) to give **15**-acetonide (179 mg, 82%). C₂₃H₂₈N₆O₅; colorless oil; TLC (EtOAc/hexane (4:1)) *R_{f}=* 0.13; [α]²²_{D} = -32.0 (EtOAc, *c* = 1.0); IR *ν*ₘₐₓ (neat) 3347, 3103, 2982, 2933, 1732, 1667, 1615, 1516, 1479, 1461, 1376, 1332, 1295, 1245, 1212, 1154, 1088 cm⁻¹; ¹H NMR (CDCl₃, 400 MHz) δ 9.01 (1 H, br s), 8.35 (1H, br s), 7.81 (1H, s), 7.14 (1H, br s), 7.04 (2 H, d, *J* = 8.0 Hz), 6.68 (2 H, d, *J* = 8.0 Hz), 6.49 (1 H, t, 4.8 Hz), 6.03 (1 H, d, *J* = 2.4 Hz), 5.33-5.38 (2 H, m), 4.70 (3 H, s), 3.09-3.16 (2 H, m), 1.62 (3 H, s), 1.37 (3 H, s), 0.90 (3 H, t, *J* = 7.2 Hz); ¹³C NMR (CDCl₃, 100 MHz) δ 168.7, 155.8, 154.2, 153.1, 147.7, 139.1, 128.8 (3 ×), 119.6, 115.4 (2 ×), 114.3, 91.6, 85.6, 83 .3, 82.4, 43.9, 34.0, 27.0, 25.1, 14.2; ESI-HRMS calcd for C₂₂H₂₆N₆O₅: 455.2043, found: *m*/*z* 455.2037 [M + H]⁺.

The acetonide of **15** (26 mg, 0.057 mmol) was stirred in 1 M HCl/THF(1:1, 0.3 mL) at room temperature for 16 h, and then neutralized with saturated NaHCO₃ aqueous solution. After concentration, the residue was triturated with H₂O to give the title compound **15,** which was then recrystallized from MeOH (14.65 mg, 62%). C₁₉H₂₂N₆O₅; white powder, mp 179.7-180.5 °C; TLC (EtOAc) *R_{f}* = 0.04; [α]²³_{D} = -27.7 (MeOH, *c* =1.0); IR *ν*ₘₐₓ (neat) 3256, 2688, 2360, 1618, 1515, 1335, 1294, 1232, 1128, 1052 cm⁻¹; ¹H NMR (CD₃OD, 400 MHz) δ 8.29 (1 H, s), 8.22 (1H, s), 7.20 (2 H, d, *J* = 8.4 Hz), 6.73 (2 H, d, *J* = 8.4 Hz), 6.00 (1 H, d, *J* = 7.6 Hz), 4.76-4.73 (1 H, m), 4.70 (2 H, br s), 4.46 (1 H, s), 4.30-4.31 (1 H, m), 3.36 (2 H, q, 7.2 Hz), 1.21 (3 H, t, 7.2 Hz); ¹³C NMR (CD₃OD, 100 MHz) δ 171.8, 157.5, 155.8, 153.6, 149.1, 141.9, 130.5, 129.9 (2 ×), 121.3, 116.2 (2 ×), 90.5, 86.3, 74.9, 73.4, 44.9, 35.2, 15.3; ESI-HRMS calcd for C₁₉H₂₁N₆O₅: 413.1573, found: *m*/*z* 413.1573 [M - H]⁺; Anal. Calcd for C₁₉H₂₂N₆O₅●H₂O: C, 52.77; H, 5.59; N, 19.43; found: C, 52.88; H, 5.40; N, 19.44.

**2-[6-(4-Methoxybenzylamino)-purin-9-yl]-5-(1*H*-tetrazol-5 -yl)-tetrahydrofuran-3,4-diol (16).** The crude acid **18** obtained from oxidation of **17**-acetonide (ca. 3.98 mmol) with PhI(OAc)₂/TEMPO was treated with ammonium chloride (426 mg, 7.96 mmol), benzotriazol-1-yl-oxytripyrrolidinophosphonium hexafluorophosphate (PyBOP, 3.07 g, 5.97 mmol), hydroxybenzotriazole (HOBt, 807 mg, 5.97 mmol) and diisopropylethylamine (2.5 mL, 15.9 mmol) in anhydrous DMF (40 mL) at 50 °C for 14 h. The mixture was concentrated under reduced pressure. The residue was extracted with CH₂Cl₂ and H₂O. The organic layer was dried over MgSO₄, filtered, concentrated, and purified by flash chromatography (silica gel; EtOAc/hexane (1:1 to 4:1)) to yield an amide product **20** as colorless oil.

A solution of dimethyl sulfoxide (0.85 mL, 11.9 mmol) in CH₂Cl₂ (10 mL) was added to a solution of oxalyl chloride (0.7 mL, 7.96 mmol) in CH₂Cl₂ (10 mL) at -78 °C. The mixture was stirred for 30 min, and a solution of amide **20** (ca. 3.98 mmol) in CH₂Cl₂ (20 mL) was added. The mixture was stirred at -78 °C for another 30 min, and diisopropylethylamine (2.6 mL, 15.9 mmol) was added. After 1 h stirring, formation of nitrile **21** was monitored by TLC analysis. The mixture was extracted with CH₂Cl₂ and H₂O. The organic phase was dried over MgSO₄, filtered, concentrated, and purified by flash chromatography (silica gel; EtOAc/hexane (2:3)) to yield nitrile **21** as colorless oil (863 mg) contaminated with a small amount of HOBt.

The above-prepared nitrile product (863 mg, 2.68 mmol) was treated with 4-methoxybenzylamine (1.84 g, 13.4 mmol) and diisopropylethylamine (15.5 mL) in 1-propanol (26 mL) at 70 °C for 4 h. The mixture was concentrated under reduced pressure, and the residue was extracted with CH₂Cl₂ and H₂O. The organic layer was dried over MgSO₄ filtered, concentrated, and purified by flash chromatography (silica gel; CH₂Cl₂/MeOH (300:1 to 150:1)) to give compound **22** (905 mg, 54% overall yield). C₂₁H₂₂N₆O₄; colorless oil; TLC (EtOAc/hexane (4:1)) *R_{f}* = 0.55; [α]²⁶_{D} = +25.8 (EtOAc, *c* = 1.0); IR *ν*ₘₐₓ (neat) 3373, 3282, 2990, 2925, 2853, 1679, 1618, 1512, 1465, 1376, 1331, 1295, 1249, 1212, 1135, 1086 cm⁻¹; ¹H NMR (CDCl₃, 400 MHz) δ 8.39 (1 H, br s), 7.64 (1 H, br s), 7.26 (2 H, d, *J* = 10.4 Hz), 6.83 (2 H, d, *J* = 10.4 Hz), 6.54 (1 H, t, *J* = 5.6 Hz), 6.13 (1H, s), 5.77 (1 H, d, *J* = 4.0 Hz), 5.68 (1 H, dd, *J* = 1.6, 4.0 Hz), 4.95 (1 H, d, *J* = 1.6 Hz), 4.75 (2 H, br s), 3.79 (3 H, s), 1.57 (3 H, s), 1.42 (3 H, s) ; ¹³C NMR (CDCl₃, 100 MHz) δ 158.8, 154.5, 153.2, 148.2, 138.9, 130.2, 128.9 (2 ×) , 119.7, 115.9, 114.5, 113.9 (2 ×), 91.6, 84.6, 83.9, 75.1, 55.3, 44.0, 26.6, 25.1; ESI-HRMS (negative mode) calcd for C₂₁H₂₂N₆O₄: 421.1624, found: *m*/*z* 421.1612 [M - H]⁻.

A solution of nitrile **22** (905 mg, 2.14 mmol) and NH₄Cl (429 mg, 8.04 mmol) in DMF (20 mL) was cooled to 0 °C, and added NaN₃ (523 mg, 8.04 mmol). The ice bath was removed; the mixture was heated to 40 °C for 1 h, slowly to 90 °C, and kept stirring at 90 °C for 9 h. The mixture was cooled, concentrated under reduced pressure, dissolved in EtOAc, and extracted with NaHCO₃ aqueous solution (pH = 8) . The combined aqueous phase was acidified by addition of HCl solution (1 M) until pH = 2, and extracted with CH₂Cl₂. The organic layer was dried over MgSO₄, filtered, and concentrated to give a practically pure tetrazole product **16**-acetonide as colorless oil (460 mg, 46% yield) . C₂₁H₂₃N₉O₄ TLC (CH₂Cl₂/MeOH (9:1)) *R_{f}* = 0.25; [α]²⁷_{D} = +13 .2 (EtOAc, *c* = 1.0) ; IR *ν*ₘₐₓ (neat) 3361, 2926, 2852, 1613, 1513, 1481, 1375, 1333, 1293, 1249, 1210, 1176, 1154, 1101, 1034 cm⁻¹; ¹H NMR (CDCl₃, 400 MHz) δ 7.90 (1 H, br s), 7.68 (1 H, br s), 7.35 (2 H, d, *J* = 8.4 Hz), 6.86 (2 H, d, *J* = 8.4 Hz), 6.83 (1 H, br s), 6.18 (1 H, s), 5.85 (1 H, s), 5.73 (1 H, d, *J* = 6.0 Hz), 5.49 (1 H, d, *J* = 6.0 Hz), 4.92 (1 H, dd, *J* = 6.8, 7.6 Hz), 4.39 (1 H, dd, *J* = 4.0, 10.4 Hz), 3.77 (3 H, s), 1.69 (3 H, s), 1.43 (3 H, s) ; ¹³C NMR (CDCl₃, 100 MHz) δ 158.4, 154.9, 152.9, 152.6, 146.2, 138.5, 129.5, 129.2 (2 ×), 118 .4, 114.2, 113.7 (2 ×), 93.4, 85.9, 83.7, 82.3, 55.4, 44.1, 27.1, 25.2; ESI-HRMS (negative mode) calcd for C₂₁H₂₃N₉O₄: 464.1795, found: *m*/*z* 1786 [M - H]⁻.

Compound **16**-acetonide (460 mg, 0.99 mmol) was stirred in 3 M HCl/THF (1:1, 0.1 mL) at room temperature for 14 h, and then neutralized with saturated NaHCO₃ aqueous solution. The mixture was concentrated under reduced pressure; the residue was taken up with THF, filtered, and concentrated to give the title compound **16** (320 mg, 76%). The purity of product was 99% as shown by HPLC on an HC-C18 column (Agilent, 4.6 × 250 mm, 5 µm) with elution of gradients of 30-60% aqueous CH₃CN in 20 min. C₁₈H₁₉N₉O₄; white powder; mp 210.0-210.6 °C; TLC (CH₂Cl₂/MeOH (9:1)) *R_{f}* = 0.05; [α]²⁶_{D} = -25.8 (THF, *c* = 1.0) ; IR *ν*ₘₐₓ (neat) 3397, 2841, 2692, 1623, 1511, 1475, 1419, 1339, 1302, 1236, 1180, 1124, 1045 cm⁻¹; ¹H NMR (DMSO-*d*₆, 400 MHz) 5 8.82 (1 H, s), 8.30 (1 H, br s), 8.20 (1H, s), 7.26 (2 H, d, *J* = 8.0 Hz), 6.83 (2 H, d, *J* = 8.0 Hz), 6.08 (1 H, d, *J* = 5.6 Hz), 5.53 (1 H, d, *J* = 6.0 Hz), 5.46 (1H, d, *J* = 2.8 Hz), 5.18 (1 H, s), 4.91 (1 H, d, *J* = 5.2 Hz), 4.62 (2 H, br s), 4.20 (1 H, s), 3.69 (3 H, d, *J* = 2.0 Hz); ¹³C NMR (DMSO-*d*₆, 100 MHz) δ 159.9, 157.4, 153.7, 151.9, 138.8, 131.5, 127.9 (2 ×), 113.1 (2 ×), 85.9, 79.1, 75.4, 74.3, 54.6, 41.9; ESI-HRMS (negative mode) calcd for C₁₈H₁₉N₉O₄: 427.1730, found: *m*/*z* 427.1727 [M - H]⁻.

**6**-**Chloro**-**9**-(**2**',**3**'-***O***-**isopropylidene**-**β**-**D**-**ribofuranosyl**)-**purine (17-aectonide).** A mixture of 6-chloropurine riboside (286 mg, 1.0 mmol), 2,2-dimethoxypropane (0.6 mL, 4.90 mmol) and p-toluenesulfonic acid monohydrate (420 mg, 2.21 mmol) in anhydrous acetone (10 mL) heated at 55 °C for 2 h. The solution was poured slowly into a stirring saturated NaHC_{O3} (10 mL), and extracted with C_{H2}Cₗ₂. The combined organic layer was dried over anhydrous MgS_{O4}, concentrated by rotary evaporation under reduced pressure, and purified by flash chromatography (silica gel; EtOAc/hexane (1:1)) to yield the title compound **17**-acetonide (264 mg, 81%). _{C13H15}Cl_{N4O4}; white powder; mp 157.4-158.8 °C; TLC (EtOAc/hexane (1:1)*) _{Rf}* = 0.23; ¹H NMR (CDCₗ₃, 400 MHz) δ 8.74 (1 H, s), 8.26 (1H, s), 5.98 (1 H, d, *J* = 4.4 Hz), 5.17 (1 H, t, *J* = 5.2 Hz), 5.08 (1 H, d, *J* = 6.0 Hz), 4.98 (1 H, br s), 4.53 (1 H, s), 3.95 (1 H, m), 3.81 (1 H, m), 1.63 (3 H, s), 1.36 (3 H, s); ¹³C NMR (CDCₗ₃, 400 MHz) δ 151.7, 151.4, 150.2, 144.5, 132.8, 114.4, 94.0, 86.6, 83.7, 81.8, 63.5, 28.1, 25.8; ESI-HRMS calcd for _{C13H16}Cl_{N4O4}: 327.0860, found: *m*/*z* 327.0913 [M+ H]⁺.

***N*⁶-(4-Dimethytaminobenzyl)adenosine (23).** By a procedure similar to that for compound **1,** 6-chloropurine riboside (100 mg, 0.35 mmol) was treated with 4-dimethylaminobenzylamine hydrochloride (5 equiv) and diisopropylethylamine (2 mL, 12 mmol) in 1-propanol (20 mL) at 70 °C for 7 h to give the title compound **23** (68 mg, 49%) after recrystallization from MeOH. C₁₉H₂₄N₆O₄; white powder; mp 170.2-170.7 °C; [α]²⁰_{D} = -64.5 (DMSO, 1 = 10 cm); TLC (MeOH/EtOAc (1:9)) *R_{f}* = 0.58; ¹NMR (DMSO-*d*₆*,* 400 MHz) δ 8.34 (1 H, s), 8.28 (1 H, br s), 8.19 (1 H, br s), 7.15 (2 H, d, *J* = 8.8 Hz), 6.63 (2 H, d, *J* = 8.8 Hz), 5.87 (1 H, d, *J* = 6.0 Hz), 5.44-5.40 (2 H, m), 5.18 (1 H, d, *J* = 4.4 Hz), 4.62-4.58 (3 H, m), 4.14 (1 H, dd, *J* = 4.4, 8.0 Hz), 3.95 (1 H, d, *J* = 4.4 Hz), 3.68-3.64 (1 H, m), 3.57-3.54 (1 H, m), 2.81 (6 H, s); ¹³C NMR (DMSO-*d*₆, 400 MHz) δ 154.2, 152.1, 149.2, 148.1, 139.6, 128.0 (2 ×), 127.3, 119.6, 112.2 (2 ×), 87.9, 85.8, 73.4, 70.6, 61.6, 42.4, 40.3 (2 ×); ESI-MS calcd for C₁₉H₂₅N₆O₄: 401.1932, found: *m*/*z* 401.1964 (M⁺ + H).

***N*⁶-(1-Pyrenylmethyl)adenosine (24).** In a round bottom flask (10 mL) were added 6-chloropurine riboside (70 mg, 0.25 mmol) and 1-pyrenemethylamine hydrochloride (167 mg, 0.63 mmol) in EtOH (3 mL). Diisopropylethylamine (0.24 mL, 2.88 mmol) was added as the base. The round bottom flask was placed into the cavity of a focused monomode microwave reactor (CEM Discover) and irradiated in 150 W for 10 min at the boiling point of EtOH. The solvent was removed by rotavapor and the residue was purified by flash chromatography (silica gel; EtOAc/MeOH (95:5)) to yield the desired product **24** (59 mg, 49%). C₂₇H₂₃N₅O₄; yellow powder; mp 143.9-144.4 °C; [α]²⁰_{D} = -50.4 (DMSO, *c* = 1.0); TLC (EtOAc/MeOH (9:1)) *R_{f}* = 0.43; ¹H NMR (DMSO-*d*₆, 400 MHz) δ 8.68 (1 H, br s), 8.54 (1 H, d, *J* = 9.2 Hz), 8.39 (1 H, s), 8.28 (2 H, d, *J* = 7.6 Hz), 8.25-8.19 (4 H, m), 5.91 (1 H, d, *J* = 6.0 Hz), 5.46 (2 H, br s), 5.40 (1 H, br s), 5.21 (1 H, br s), 4.63 (1 H, d, *J* = 4.4 Hz), 4.16 (1 H, br s), 3.98 (1 H, d*, J* = 2.8 Hz), 3.68 (1 H, d, *J* = 12 Hz), 3.58 (1 H, m); ¹³C NMR (DMSO-*d*₆, 100 MHz) δ 151.8, 139.5, 130.3, 129.8, 129.3, 127.3, 126.9, 126.8, 126.3, 125.7, 124.7, 124.6, 124.2, 123.5, 123.4, 122.7, 87.5, 85.5, 73.1, 70.2, 61.3; ESI-HRMS calcd. for C₂₇H₂₄N₅O₄: 482.1823, found: *m*/*z* 482.1827 [M + H]⁺.

**Radioligand binding assays.** Radioligand binding assays were performed by MDS Pharma Services Taiwan (Taipei, Taiwan) using standard binding protocols. For the binding assay of the A_{2A} receptor, ⁴⁶ membrane proteins collected from HEK293 cells overexpressing the human A_{2A} receptor were incubated in reaction buffer [50 mM Tris-HCl (pH 7.4), 10 mM MgCl₂, 1 mM EDTA, and 2 U/mL adenosine deaminase] containing ³H-CGS21680 (50 nM) for 90 min at 25 °C. Nonspecific binding was assessed in the presence of 50 µM adenosine-5'-*N*-ethylcarboxamide (NECA).

Binding assays for adenosine transporters were conducted as described earlier.⁴⁷ Membrane fractions collected from the cerebral cortex of Duncan Hartley derived guinea pigs were incubated with ³H-labeled NBTI (0.5 nM) for 30 min at 25 °C in an incubation buffer containing 50 mM Tris-HCl (pH 7.4). Nonspecific binding was assessed in the presence of 5 µM NBTI, a high-affinity inhibitor of equilibrative nucleoside transporter 1 (ENT1), which inhibits only ENT1 at 0.5 nM.⁴⁸ Reactions were terminated by filtration over GF/B glass fibers and washing with the corresponding reaction buffer.

**MTT metabolism assay.** PC12 cells purchased from ATCC (Manassas, VA, USA) were maintained in Dulbecco's modified Eagle's medium (DMEM) supplemented with 10% horse serum and 5% fetal bovine serum and incubated in a CO₂ incubator (5%) at 37 °C. Survival was assessed by the 3-(4,5-dimethylthiazol-2-yl)-2,5-diphenyltetrazolium bromide (MTT) metabolism assay as described elsewhere.^{49, 50} In brief, cells grown on 150-mm plates were washed three times with PBS and resuspended in DMEM. Suspended cells (1 × 10⁴ cells) were plated on 96-well plates and treated with or without the indicated reagent. After incubation for 24 h, MTT (0.5 mg/mL) was added to the medium and incubated for 3 h. After discarding the medium, DMSO (100 µL) was then applied to the well to dissolve the formazan crystals derived from the mitochondrial cleavage of the tetrazolium ring by live cells. The absorbance at 570/630 nm in each well was measured on a micro-enzyme-linked immunosorbent assay (ELISA) reader.

### Citation List:

1. Andrew, S. E.; Goldberg, Y. P.; Kremer, B.; Telenius, H.; Theilmann, J.; Adam, S.; Starr, E.; Squitieri, F.; Lin, B.; Kalchman, M. A. ; Graham, R. K.; Hayden, M. R. The relationship between trinucleotide (CAG) repeat length and clinical features of Huntington's disease. Nat. Genet. 1993, 4, 398-403.
2. DiFiglia, M.; Sapp, E.; Chase, K. O.; Davies, S. W.; Bates, G. P.; Vonsattel, J. P.; Aronin, N. Aggregation of huntingtin in neuronal intranuclear inclusions and dystrophic neurites in brain. Science 1997, 277, 1990-1993.
3. MacDonald, M. E.; Ambrose, C. M. ; Duyao, M. P.; Myers, R. H.; Lin, C.; Srinidhi, L.; Barnes, G.; Taylor, S. A.; James, M.; Groot, N. A novel gene containing a trinucleotide repeat that is expanded and unstable on Huntington's disease chromosomes. Cell 1993, 72, 971-983.
4. Beal, M. F. ; Ferrante, R. J. Experimental therapeutics in transgenic mouse models of Huntington's disease. Nat. Rev. Neurosci. 2004, 5, 373-384.
5. Okamoto, S. -i.; Pouladi, M. A.; Talantova, M.; Yao, D.; Xia, P.; Dagmar Ehrnhoefer. E.; Zaidi, R.; Clemente, A.; Kaul, M. ; Graham, R. K. ; Zhang, D. ; Chen, H. -S. V.; Tong, G.; Hayden, M. R. ; Lipton, S. A. Balance between synaptic versus extrasynaptic NMDA receptor activity influences inclusions and neurotoxicity of mutant huntingtin. Nat. Med. 2009, 15, 1407-1413.
6. Chou, S. Y.; Lee, Y. C.; Chen, H. M.; Chiang, M. C.; Lai, H. L.; Chang, H. H. ; Wu, Y. C.; Sun, C. N. ; Chien, C. L . ; Lin, Y. S.; Wang, S. C.; Tung, Y. Y.; Chang , C.; Chern, Y. CGS21680 attenuates symptoms of Huntingtin's disease in a transgenic mouse model. J. Neurochem. 2005, 93, 310-320.
7. Chiang, M. C.; Chen, H. M.; Lai, H. L.; Chen, H. W.; Chou, S. Y.; Chen, C. M.; Tsai, F. J.; Chern, Y. The A2A adenosine receptor rescues the urea cycle deficiency of Huntington's disease by enhancing the activity of the ubiquitin-proteasome system. Hum. Mol. Genet. 2009, 18, 2929-2942.
8. Link, A. A.; Kino, T.; Worth, J. A.; McGuire, J. L.; Crane, M. L. ; Chrousos, G. P.; Wilder, R. L. ; Elenkov, I. J. Ligand-activation of the adenosine A2A receptors inhibits IL-12 production by human monocytes. J. Immunol. 2000, 164, 436-442.
9. Huang, N.-K.; Chern, Y. ; Fang, J.-M.; Lin, C.-I; Chen, W.-P.; Lin, Y.-L. Neuroprotective principles from Gastrodia elate. J. Nat. Prod. 2007, 70, 571-574.
10. Fink, J. S.; Weaver, D. R; Rivkees, S. A; Peterfreund, R. A.; Pollack, A. E.; Adler, E. M.; Reppert, S. M. Molecular cloning of the rat A2 adenosine receptor: selective co-expression with D2 dopamine receptors in rat striatum. Brain Res. Mol. Brain Res. 1992, 14, 186-195.
11. Dixon, A. K.; Gubitz, A. K. ; Sirinathsinghji, D. J. S.; Richardson, P. J.; Freeman, T. C. Tissue distribution of adenosine receptor mRNAs in the rat. Br. J. Pharmacol . 1996, 118, 1461-1468.
12. Rosin, D. L.; Robeva, A.; Woodard, R. L.; Guyenet, P. G.; Linden, J. Immunohistochemical localization of adenosine A2A receptors in the rat central nervous system. J. Comp. Neurol. 1998, 401, 163-186.
13. Anderson, C. M.; Xiong, W.; Geiger, J. D.; Young, J. D.; Cass, C. E.; Baldwin, S. A.; Parkinson, F. E. Distribution of equilibrative, nitrobenzylthioinosine-sensitive nucleoside transporters (ENT1) in brain. J. Neurochem. 1999, 73, 867-873.
14. Sapp, E.; Schwarz, C.; Chase, K. ; Bhide, P. G. ; Young, A. B.; Penney, J.; Vonsattel, J. P.; Aronin, N.; DiFiglia, M. Huntingtin localization in brains of normal and Huntington's disease patients. Ann. Neurol. 1997, 42, 604-612.
15. Ohlson, S. Designing transient binding drugs: A new concept for drug discovery. Drug Discov. Today 2008, 13, 433-439.
16. Morphy, R.; Kay, C.; Rankovic, Z. From magic bullets to designed multiple ligands. Drug Discov. Today 2004, 9, 641-651.
17. Morphy, R.; Rankovic, Z. Designed multiple ligands. an emerging drug discovery paradigm. J. Med. Chem. 2005, 48, 6523-6543.
18. Zimmermann, G. R. ; Lehar, J.; Keith, C. T. Multi-target therapeutics: when the whole is greater than the sum of the parts. Drug Discov. Today 2007, 12, 34-42.
19. Hopkins, A. L. Network pharmacology: the next paradigm in drug discovery. Nat. Chem. Bio. 2008, 4, 682-690.
20. Popoli, P.; Blum, D.; Domenici, M. R.; Burnouf, S.; Chern, Y. A critical evaluation of adenosine A2A receptors as potentially "druggable" targets in Huntington's disease. Curr. Pharm. Des. 2008, 14, 1500-1511.
21. Morphy, R.; Rankovic, Z. The Physicochemical challenges of designing multiple ligands. J. Med. Chem. 2006, 49, 4961-4970.
22. Morphy, R.; Rankovic, Z. Designing multiple ligands - medicinal chemistry strategies and challenges. Curr. Pharm. Des. 2009, 15, 587-600.
23. Kurogi, Y.; Güner, O. Pharmacophore modeling and three-dimensional database searching for drug design using catalyst. Curr. Med. Chem. 2001, 8, 1035-1055.
24. Kaminski, J. J.; Rane, D. F.; Snow, M. E.; Weber, L.; Rothofsky, M. L.; Anderson, S. D.; Lin, S. L. Identification of novel farnesyl protein transferase inhibitors using three-dimensional database searching methods. J. Med. Chem. 1997, 40, 4103-4112.
25. Singh, J.; Van Vlijmen, H.; Liao, Y.; Lee, W. C.; Cornebise, M. ; Harris, M. ; Shu, I. H. ; Gill, A. ; Cuervo, J. H.; Abraham, W. M.; Adams, S. P. Identification of potent and novel α4β1 antagonists using in silico screening. J. Med. Chem. 2002, 45, 2988-2993.
26. Kotsikorou, E.; Oldfield, E. A quantitative structure-activity relationship and pharmacophore modeling investigation of aryl-X and heterocyclic bisphosphonates as bone resorption agents. J. Med. Chem. 2003, 46, 2932-2944.
27. Chen, G. S.; Chang, C. S.; Kan, W. M.; Chang, C. L.; Wang, K. C. ; Chern, J. W. Novel lead generation through hypothetical pharmacophore three-dimensional database searching: discovery of isoflavonoids as nonsteroidal inhibitors of rat 5α-reductase. J. Med. Chem. 2001, 44, 3759-3763.
28. Zampieri, D.; Mamolo, M. G.; Laurini, E.; Florio, C.; Zanette, C. ; Fermeglia, M. ; Posocco, P. ; Paneni, M. S.; Pricl, S.; Vio, L. Synthesis, biological evaluation, and three-dimensional in silico pharmacophore model for σ1 receptor ligands based on a series of substituted benzo[d]oxazol-2(3H)-one derivatives. J. Med. Chem. 2009, 52, 5380-5393.
29. Wei, D.; Jiang, X.; Zhou, L.; Chen, J.; Chen, Z.; He, C.; Yang, K.; Liu, Y.; Pei, J.; Lai, L. Discovery of multitarget inhibitors by combining molecular docking with common pharmacophore matching. J. Med. Chem. 2008, 51, 7882-7888.
30. Jaakola, V. P. ; Griffith, M. T. ; Hanson, M. A. ; Cherezov, V. ; Chien, E. Y. ; Lane, J. R. ; Ijzerman, A. P. ; Stevens, R. C. The 2.6 angstrom crystal structure of a human A2A adenosine receptor bound to an antagonist. Science 2008, 322, 1211-1217.
31. Walkinshaw, G. & Waters, C.M. Neurotoxin-induced cell-death in neuronal pc12 cells is mediated by induction of apoptosis. Neuroscience 1994, 63, 975-987.
32. Chiang, M. C.; Lee, Y. C.; Huang, C. L.; Chern, Y. cAMP-response element-binding protein contributes to suppression of the A2A adenosine receptor promoter by mutant Huntingtin with expanded polyglutamine residues. J. Biol. Chem. 2005, 280, 1.4331-14340.
33. Jarvis, M. F.; Schulz, R.; Hutchison, A. J.; Do, U. H. ; Sills, M. A.; Williams, M. [3H] CGS 21680, a selective A2 adenosine receptor agonist directly labels A2 receptors in rat brain. J. Pharmacol. Exp. Ther. 1989, 251, 888-893.
34. Catalyst, Accelrys, 9685 North Scranton Road, San Diego, CA 92121, U.S.A. http://www.accelrys.com
35. Cass, C. E.; Gaudette, L. A.; Paterson, A. R. Mediated transport of nucleosides in human erythrocytes - specific binding of inhibitor nitrobenzylthioinosine to nucleoside transport sites in erythrocyte-membrane. Biochim. Biophys. Acta 1974, 345, 1-10.
36. Zhu, Z. X.; Buolamwini, J. K. Constrained NBMPR analogue synthesis, pharmacophore mapping and 3D-QSAR modeling of equilibrative nucleoside transporter 1 (ENT1) inhibitory activity. Bioorg. Med. Chem. 2008, 16, 3848-3865.
37. Dixon, S. L.; Smondyrev, A. M.; Knoll, E. H.; Rao, S. N.; Shaw, D. E.; Friesner, R. A. PHASE: a new engine for pharmacophore perception, 3D QSAR model development, and 3D database screening: 1. Methodology and preliminary results. J. Comput. Aided Mol. Des. 2006, 20, 647-671.
38. Kolb, H. C.; Finn, M. G.; Sharpless, K. B. Click chemistry: diverse chemical function from a few good reactions. Angew. Chem. Int. Ed. 2001, 40, 2004-2021.
39. Epp, J. B.; Widlanski, T. S. Facile preparation of nucleoside-5'-carboxylic acids. J. Org. Chem. 1999, 64, 293-295.
40. Nakajima, N. ; Saito, M. ; Ubukata, M. Activated dimethyl sulfoxide dehydration of amide and its application to one-pot preparation of benzyl-type perfluoroimidates. Tetrahedron, 2002, 58, 3561-3577.
41. Bosch, M. P.; Campos, F.; Niubó, I . ; Rosell, G.; Díaz, J. L.; Brea, J.; Loza, M. I.; Guerrero, A. Synthesis and biological activity of new potential agonists for human adenosine A2A receptor. J. Med. Chem. 2004, 47, 4041-4053.
42. Martin, P. L.; Barrett, R. J.; Linden, J.; Abraham, W. M. Pharmacology of 2-cyclohexylmethylidenehydrazinoadenosine (WRC-0470), a novel, short-acting adenosine A2A receptor agonist that produces selective coronary vasodilation. Drug Develop. Res. 1997, 40, 313-324.
43. Volpini, R.; Camaioni, E.; Costanzi, S.; Vittori, S.; Klotz, K. N.; Cristalli, G. Synthesis of di- and tri-substituted adenosine derivatives and their affinities at human adenosine receptor subtypes. Nucleosides Nucleotides 1999, 18, 2511-2520.
44. Vittori, S.; Costanzi, S.; Lambertucci, C.; Portino, F. R. ; Taffi, S.; Volpini, R. ; Klotz, K. N. ; Cristalli, G. A2B adenosine receptor agonists: synthesis and biological evaluation of 2-phenylhydroxypropynyl adenosine and NECA derivatives. Nucleosides, Nucleotides Nucleic Acids 2004, 23, 471-481.
45. Güner, O.; Clement, O.; Kurogi, Y. Pharmacophore modeling and three dimensional database searching for drug design using catalyst: recent advances. Curr. Med. Chem. 2004, 11, 2991-3005.
46. Varani, K.; Gessi, S.; Dalpiaz, A.; Borea, P. A. Pharmacological and biochemical characterization of purified A2A adenosine receptors in human platelet membranes by [3H] -CGS 21680 binding. Br. J. Pharmacol. 1996, 117, 1693-701.
47. Verma, A. & Marangos, P.J. Nitrobenzylthioinosine binding in brain: an interspecies study. Life Sci. 1985, 36, 283-290.
48. Ward, J. L.; Sherali, A.; Mo, Z. P.; Tse, C. M. Kinetic and pharmacological properties of cloned human equilibrative nucleoside transporters, ENT1 and ENT2, stably expressed in nucleoside transporter-deficient PK15 Cells. Ent2 exhibits a low affinity for guanosine and cytidine but a high affinity for inosine. J. Biol. Chem. 2000, 275, 8375-8381.
49. Mosmann, T. Rapid colorimetric assay for cellular growth and survival: application to proliferation and cytotoxicity assays. J. Immunol. Methods. 1983, 65, 55-63.
50. Huang, N. K.; Lin, Y. W. ; Huang, C. L. ; Messing, R. O. ; Chern, Y. Activation of protein kinase A and a typical protein kinase C by A2A adenosine receptors antagonizes apoptosis due to serum deprivation in PC12 cells. J. Biol. Chem. 2001, 276, 13838-13846.

## Claims

1. A composition comprising an effective amount of a compound of formula: 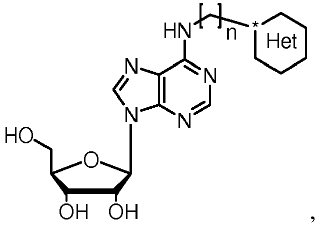 wherein n is 1 to 3, the (substituted) heterocycle contains 5- or 6-membered rings and the fused heterocycle contains nitrogen, oxygen or sulfur heteroatoms and the substituent is selected from the group consisting of hydrogen, fluorine, chlorine, bromine, iodine, hydroxy, C₁₋₆ alkyl, trifluoromethyl and (substituted) phenyl, for use in treating a neurodegenerative disease in a subject in need thereof.

2. The composition of claim 1, wherein the compound is

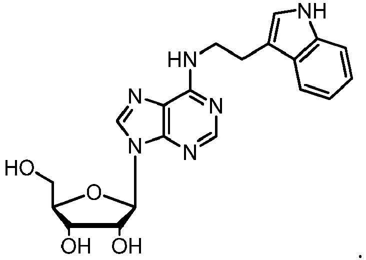

3. The composition of claim 1 or 2, wherein the neurodegenerative disease is a protein-misfolding disease.

4. The composition of claim 1 or 2, wherein the neurodegenerative disease is selected from the group consisting of Alzheimer's disease, Parkinson's disease, amyotrophic lateral sclerosis, Prion disease, Huntington's disease, and spinal cerebellar ataxias.

5. The composition of claim 4, wherein the neurodegenerative disease is Huntington's disease.

6. The composition of claim 4, wherein the neurodegenerative disease is Alzheimer's disease.

7. The composition of claim 4, wherein the neurodegenerative disease is amyotrophic lateral sclerosis.

8. The composition of claim 4, wherein the neurodegenerative disease is spinal cerebellar ataxias.

## Patentansprüche

1. Zusammensetzung, umfassend eine wirksame Menge einer Verbindung der folgenden Formel: 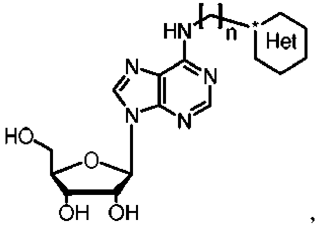 wobei n 1 bis 3 ist, der (substituierte) Heterocyclus 5- oder 6-gliedrige Ringe enthält und der fusionierte Heterocyclus Stickstoff-, Sauerstoff- oder Schwefelheteroatome enthält und der Substituent aus der Gruppe ausgewählt ist, die aus Wasserstoff, Fluor, Chlor, Brom, Iod, Hydroxy, C₁₋₆-Alkyl, Trifluormethyl und (substituiertem) Phenyl besteht, zur Verwendung bei der Behandlung einer neurodegenerativen Erkrankung bei einem Subjekt, das dieser bedarf.

2. Zusammensetzung nach Anspruch 1, wobei die Verbindung folgende ist:

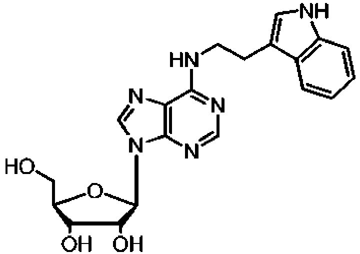

3. Zusammensetzung nach Anspruch 1 oder 2, wobei die neurodegenerative Erkrankung eine Proteinfehlfaltungserkrankung ist.

4. Zusammensetzung nach Anspruch 1 oder 2, wobei die neurodegenerative Erkrankung aus der Gruppe ausgewählt ist, die aus Alzheimer-Krankheit, Parkinson-Krankheit, amyotropher Lateralsklerose, Prionenkrankheit, Chorea Huntington und spinaler zerebellärer Ataxie besteht.

5. Zusammensetzung nach Anspruch 4, wobei die neurodegenerative Erkrankung Chorea Huntington ist.

6. Zusammensetzung nach Anspruch 4, wobei die neurodegenerative Erkrankung Alzheimer-Krankheit ist.

7. Zusammensetzung nach Anspruch 4, wobei die neurodegenerative Erkrankung amyotrophe Lateralsklerose ist.

8. Zusammensetzung nach Anspruch 4, wobei die neurodegenerative Erkrankung spinale zerebelläre Ataxie ist.

## Revendications

1. Composition comprenant une quantité efficace d'un composé de formule : 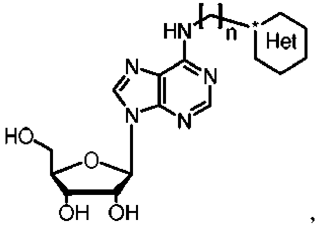 dans laquelle n vaut 1 à 3, l'hétérocycle (substitué) contient des cycles de 5 à 6 chaînons et l'hétérocycle fusionné contient des hétéroatomes d'azote, d'oxygène ou de soufre et le substituant est choisi dans le groupe constitué par un atome d'hydrogène, de fluor, de chlore, de brome, d'iode, un groupe hydroxy, alkyle en C₁ à C₆, trifluorométhyle et phényle (substitué), destinée à être utilisée dans le traitement d'une maladie neurodégénérative chez un sujet en ayant besoin.

2. Composition selon la revendication 1, ledit composé étant

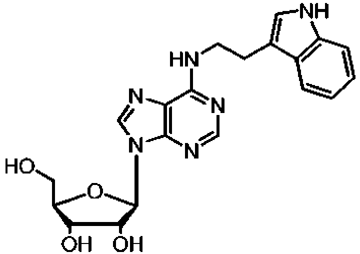

3. Composition selon la revendication 1 ou 2, ladite maladie neurodégénérative étant une maladie associée au mauvais repliement des protéines.

4. Composition selon la revendication 1 ou 2, ladite maladie neurodégénérative étant choisie dans le groupe constitué par la maladie d'Alzheimer, la maladie de Parkinson, la sclérose latérale amyotrophique, une maladie à prions, la maladie d'Huntington et les ataxies spinocérébelleuses.

5. Composition selon la revendication 4, ladite maladie neurodégénérative étant la maladie d'Huntington.

6. Composition selon la revendication 4, ladite maladie neurodégénérative étant la maladie d'Alzheimer.

7. Composition selon la revendication 4, ladite maladie neurodégénérative étant la sclérose latérale amyotrophique.

8. Composition selon la revendication 4, ladite maladie neurodégénérative étant les ataxies spinocérébelleuses.
